# EUROPÄISCHE PATENTSCHRIFT

(11) **EP 2 816 355 B1**
(45) Veröffentlichungstag und Bekanntmachung des Hinweises auf die Patenterteilung: **09.08.2017**
(21) Anmeldenummer: 14173356.8
(22) Anmeldetag: 20.06.2014
(51) Int. Cl.: G01N 33/569, C07K 14/00, G01N 33/566, C07K 14/005, C12N 15/62

(54) **Detektionsverfahren zur Detektion von Bakterien, Verfahren zur Herstellung von Fusionsproteinen und Fusionsprotein**
Detection method for detection of bacteria, method for the preparation of fusion proteins and fusion protein
Procédé de détection de bactéries, procédé de fabrication de protéines de fusion et protéine de fusion

(30) Priorität: 20.06.2013 DE 102013106462
(43) Veröffentlichungstag der Anmeldung: 24.12.2014
(73) Patentinhaber: Airbus Defence and Space GmbH, 85521 Ottobrunn (DE); Astrium GmbH, 82024 Taufkirchen (DE)
(72) Erfinder: Reidt, Ulrich, 34613 Schwalmstadt (DE); Friedberger, Alois, 85667 Oberpframmern (DE); Hummel, Thomas, 88682 Salem (DE)
(74) Vertreter: Kastel, Stefan

(56) Entgegenhaltungen:
- WO-A1-2004/088321
- WO-A2-03/000888
- WO-A2-2010/036860
- DE-A1- 10 036 931
- DE-A1- 19 837 751
- US-A1- 2002 173 006
- US-A1- 2003 170 622
- US-A1- 2006 068 421
- US-A1- 2008 096 769
- US-A1- 2009 163 368
- US-A1- 2011 318 269

## Beschreibung

Die Erfindung betrifft ein Detektionsverfahren zur Detektion von Bakterien, ein Verfahren zur Herstellung von Fusionsproteinen sowie ein mit einem solchen Verfahren erhältliches Fusionsprotein.

Gegenwärtig werden Bakterien durch folgende Methoden detektiert: Kultivierung auf Selektivnährböden, Bunte Reihe (API-Test, VITEK), Polymerase-Kettenreaktion (PCR), Enzyme Linked Immunosorbent Assay (ELISA), Fluoreszenz-in-situ-Hybridisierung (FISH), In-situ-Hybridisierung (ISH), Aptamere

Die meisten spezifischen mikrobiologischen Detektionsverfahren besitzen gewisse Nachteile, die auf dem zugrundliegenden Verfahren an sich beruhen. Zum Beispiel sind etablierte Nachweismethoden für Mikroorganismen oft sehr zeitintensiv, da die Mikroorganismen zunächst auf Nährmedien überführt und bebrütet werden müssen. Nach dieser Voranreicherung erfolgt dann eine selektive Anreicherung und anschließend ein biochemisches Screening (API-Test).

Oftmals zeigt dieser klassische Nachweisprozess falsch-positive oder falsch-negative Ergebnisse und es müssen immunologische (ELISA) und erbgutbasierende Verfahren (PCR, ISH und FISH) zusätzlich hinzugezogen werden. Diese Verfahren sind in der Routine sehr teuer und zeigen auch in Bezug auf die Identifizierung eine gewisse Unsicherheit. Zum Beispiel zeigt ein ELISA, der auf einem polyklonalen Antikörper aufbaut, oftmals Kreuzreaktionen zu anderen Organismen. Ein weiterer Nachteil dieses immunologischen Detektionsverfahrens ist die Endlichkeit der Ressource, da ein polyklonaler Antikörper durch Immunisierung eines Tieres (Hase, Maus, Ziege, Pferd) produziert wird.

WO 2004/088321 A1 beschreibt ein Anreicherungsverfahren zum Anreichern von Zielzellen durch Bindung unter Verwendung von Bindungsdomänen von Zellwänden, sowie die Verwendung eines solchen Anreicherungsverfahrens bei der Detektion von spezifischen Zellen.

WO 03/000888A2 beschreibt ein Verfahren zur selektiven Aufreinigung von Bakterienzellen und/oder Zellbestandteilen, wobei die Aufreinigung mittels eines festen Trägers durchgeführt wird

DE 100 36 931 A1 betrifft ein Verfahren zum Nachweis von Bakterien, umfassend die folgenden Schritte: Kopplung von Bakteriophagen und/oder Bakterienphagenproteinen an einen Träger, Inkubieren des mit den Bakteriophagen und/oder Bakterienphagenproteinen gekoppelten Trägers mit einer Probe, gegebenenfalls Entfernen der Probe und der nicht an die Bakteriophagen und/oder Bakterienphagenproteine gebundenen Bakterien der Probe, gegebenenfalls Zugabe von die Bakterienmembran permeabilisierenden oder zerstörenden Substanzen, und Nachweisen der an die Bakteriophagen und/oder Bakterienphagenproteine gebundenen Bakterien der Probe, wobei die gebundenen Bakterien keinem Kultivierungsschritt unterzogen werden.

DE 198 37 751 A1 betrifft die Verwendung von speziellen Polypeptiden, welche Teile (Zellwand-bindende Domänen, CBD) von Zellwand-bindenden Proteinen und/oder Enzymen darstellen. Die CBD Polypeptide oder Fusionsprodukte, enthaltend diese Polypeptide, können gezielt an die Zellwand (auch Zellmembran) von Zielzellen binden. Verfahren zur erkennbaren Markierung (insbesondere für diagnostische Verfahren), Immobilisierung, Anreicherung und Reinigung von insbesondere Mikroorganismenzellen (Bakterienzellen, Hefezellen, Schimmelzellen) sowie deren Dauerformen (Bakteriensporen, Schimmelsporen), aber auch eukaryontischen (tierischen und/oder pflanzlichen) Zellen werden offenbart. Die CBD Polypeptide können direkt markiert werden oder die Bindung in einem zweiten Schritt nach Art eines Sandwich Assays nachgewiesen werden.

WO 2010/036860 A1 offenbart ein Verfahren zum Aufreinigen eines Fusionsproteins unter Verwendung eines Reiniguns-Tags, der über eine Proteaseschnittstelle mit dem Fusionsprotein verbunden ist.

US 2009/163368 A1, US 2011/318269 A1, US 2008/096769 A1, US 2006/068421 A1 und US 2003/170622 A1 beschreiben jeweils Verfahren zur Herstellung eines Fusionsproteins, das ein Bakteriophagenprotein, ein Linkerprotein und ein Markerprotein aufweist.

Aufgabe der Erfindung ist es daher, ein verbessertes Verfahren zur Detektion von Bakterien vorzuschlagen, das die oben genannten Nachteile überwindet.

Diese Aufgabe wird mit einem Verfahren mit den Merkmalen des Anspruches 1 gelöst.

Ein Verfahren zur Herstellung von Fusionsproteinen sowie ein mit diesem Verfahren erhältliches Fusionsprotein ist Gegenstand der Nebenansprüche.

Vorteilhafte Ausgestaltungen der Erfindung sind Gegenstand der Unteransprüche.

Ein Detektionsverfahren zur Detektion von Bakterien weist die folgenden Schritte auf:
a) spezifisches Anreichern eines bakteriellen Oberflächenproteins an einer Anreicherungsstruktur;
b) spezifische Detektionsreaktion eines Detektionsproteins mit einem bakteriellen Oberflächenprotein;
c) Erfassen des Detektionsproteins,
wobei als Detektionsprotein ein Fusionsprotein verwendet wird, das durch Verbinden eines Bakteriophagenproteins mit einem Markerprotein über ein Linkerprotein hergestellt wird, wobei das Markerprotein aus der Gruppe der humanen Caspasen ausgewählt wird.

Vorzugsweise wird ein freies bakterielles Oberflächenprotein oder ein gebundenes, insbesondere ein an ein Bakterium gebundenes, bakterielles Oberflächenprotein angereichert.

Vorteilhaft wird die Anreicherungsstruktur an einer immobilen oder einer mobilen Oberflächenstruktur bereitgestellt.

Besonders bevorzugt wird die Anreicherungsstruktur über eine Linker-Struktur, insbesondere über einen Biotin-Streptavidin-Linker oder einen Metallchelat-Polyhistidin-Tag-Linker, an der Oberflächenstruktur immobilisiert.

Vorteilhaft werden als Oberflächenstruktur magnetische Beads, mehr insbesondere Ni-Beads, nicht magnetische Beads, insbesondere Ni-NTA, Chromatographiesäulenmaterialien, Sepharosen, Mikrotiterplatten, Kunststoffe, Filteroberflächen, Glas, Metall, Keramik, Holz, Gestein, Gewebe, Nanopartikel und/oder Halbleitermaterialien verwendet.

Vorteilhaft wird das Bakteriophagenprotein als freies Bakteriophagenprotein bereitgestellt.

Besonders bevorzugt wird ein für ein Bakterium oder für eine Bakterienfamilie selektives Bakteriophagenprotein verwendet.

Bevorzugt wird sowohl das Anreichern als auch die Detektionsreaktion durch spezifische Protein-Protein-Interaktion mit einem bakteriellen Oberflächenprotein durchgeführt.

Bevorzugt erfolgen Anreicherung und Detektionsreaktion an unterschiedlichen Epitopen des bakteriellen Oberflächenproteins.

Beispielsweise wird als Detektionsprotein das Fusionsprotein und als Anreicherungsstruktur ein Antikörper verwendet.

Weiter alternativ ist es auch möglich, sowohl für das Anreichern als auch für die Detektionsreaktion ein Fusionsprotein zu verwenden.

Es wird unmittelbar an eine Detektionsproteinoberfläche ein Markermolekül, insbesondere ein Enzym oder ein Fluoreszenzmolekül, angebunden.

Als Detektionsprotein wird ein Fusionsprotein verwendet, das durch Verbinden eines Bakteriophagenproteins mit einem Markerprotein über ein Linkerprotein hergestellt wird.

Vorteilhaft wird dabei das Fusionsprotein insbesondere über eine Proteaseschnittstelle mit einem Reinigungs-Tag gekoppelt.

Vorzugsweise wird das Fusionsprotein durch rekombinante Expression hergestellt.

Besonders bevorzugt wird vor Schritt a) ein Proteinherstellungsschritt mit den folgenden Schritten durchgeführt wird:
i) rekombinantes Exprimieren eines Fusionsproteins oder eines Bakteriophagenproteins in einem niederen prokaryotischen oder eukaryotischen Organismus;
ii) Aufschließen der Zellen des niederen prokaryotischen oder eukaryotischen Organismus;
iii) Bilden eines Proteinextrakts;
iv) Reinigen des Proteinextrakts.

Vorzugsweise werden die durch die Expression gebildeten Fusionsproteine stabilisiert.

Die Stabilisierung erfolgt dabei vorteilhaft durch Vakuumtrocknen, Gefriertrocknen, Lyophilisieren, Sublimationstrocknen, PEGyliseren, Protein-Engineering und/oder durch Zugeben von Stabilisatoren.

Beim PEGylieren wird das exprimierte Fusionsprotein vorteilhaft mit Polyethylenglycol-Molekülen umhüllt.

Ein Verfahren zur Herstellung von Fusionsproteinen weist die folgenden Schritte auf:
A) Klonieren eines Fusionsproteingens;
B) Transformieren des Fusionsproteingens in einen niederen prokaryotischen oder eukaryotischen Organismus;
C) Exprimieren von Fusionsproteinen in dem Organismus;
D) Reinigen der Fusionsproteine,
wobei in Schritt A) ein Bakteriophagenprotein-Gen, ein Linkerprotein-Gen und ein Markerprotein-Gen ligiert werden.

Das derart erzeugte Fusionsprotein wird in dem oben beschriebenen Detektionsverfahren zum Detektieren von Bakterien verwendet.

Als Markerproteingene werden dabei Gene zur Bildung von humanen Caspasen, insbesondere Caspase-3, als Markerproteine verwendet.

Vorzugsweise können zusätzlich als Markerproteingene Gene zur Bildung von Markerproteinen verwendet, die aus der Gruppe Enzyme, Proteine, fluoreszierende Proteine, biolumineszierende Enzyme, insbesondere Luziferase, lytische Proteine und/oder Polymerasen, insbesondere Tag-Polymerase, ausgewählt werden.

Vorteilhaft wird die Ligation in Schritt A) seriell durchgeführt.

Vorzugsweise wird dabei zuerst das Bakteriophagenprotein-Gen, insbesondere N-terminal, und dann das Markerprotein-Gen, insbesondere C-terminal, mit dem Linkerprotein-Gen ligiert.

Vorteilhaft werden für die Ligation unterschiedliche Restriktionsenzyme für das Bakteriophagenprotein-Gen und das Markerprotein-Gen verwendet.

Beispielsweise wird BsmBl als 5'-Ende und Acc65I als 3'-Ende für das Bakteriophagenprotein-Gen und Sall als 5'-Ende und Notl als 3'-Ende für das Markerprotein-Gen verwendet.

Besonders bevorzugt wird ein Linkerprotein erzeugt, das eine freie dreidimensionale Bewegung des Bakteriophagenproteins und des Markerproteins zueinander ermöglicht.

Vorteilhaft weist dazu das erzeugte Linkerprotein eine Länge von neun Codons auf und ist mehr insbesondere frei von Stopcodons.

Ein bevorzugtes Fusionsprotein ist hergestellt mit dem oben beschriebenen Verfahren.

Ausführungsbeispiele der Erfindung werden nachfolgend anhand der beigefügten Zeichnungen näher erläutert. Darin zeigt:
- Fig. 1: ein Coating über einen Polyhistidin-Tag;
- Fig. 2: die Anreicherung von Bakterien über Metallchelate und Bakteriophagenproteine;
- Fig. 3: die Anreicherung von Bakterien über Biotin-Streptavidin und Bakteriophagenproteine;
- Fig. 4: die Immobilisierung von Bakteriphagenproteinen über Biotin-Steptavidin;
- Fig. 5: die Immobilisierung von Bakteriphagenproteinen über Metallchelate;
- Fig. 6: einen nicht erfindungsgemäßen Hybrid-Phago-Immuno-Assay mit einem Antikörper als Detektionsprotein;
- Fig. 7: einen Hybrid-Phago-Immuno-Assay mit einem Bakteriophagenprotein als Detektionsprotein, wobei der Antikörper über Protein A oder Protein G immobilisiert ist;
- Fig. 8: einen Hybrid-Phago-Immuno-Assay mit einem Bakteriophagenprotein als Detektionsprotein, wobei der Antikörper über Biotin-Streptavidin immobilisiert ist;
- Fig. 9: schematische Darstellungen eines Fusionsproteins;
- Fig. 10: ein Fusionsprotein mit einem fluoreszierenden Protein als Detektorprotein;
- Fig. 11: ein Fusionsprotein mit Caspase-3 als Detektorprotein;
- Fig. 12: ein Fusionsprotein mit Luziferase als Detektorprotein;
- Fig. 13: ein Fusionsprotein mit Tag-Polymerase als Detektorprotein;
- Fig. 14: eine schematische Darstellung eines Hybrid-Phago-Immuno-Assay mit unterschiedlichen Fusionsproteinen als Detektionsproteinen, wobei der Antiköper über Protein A oder Protein G immobilisiert ist;
- Fig. 15: eine schematische Darstellung eines Hybrid-Phago-Immuno-Assay mit unterschiedlichen Fusionsproteinen als Detektionsproteinen, wobei der Antiköper über Biotin-Streptavidin immobilisiert ist;
- Fig. 16: ein Fusionsprotein mit einem lytischen Protein;
- Fig. 17: N-Acetyl-Asp-Glu-Val-Asp p-nitroanilid;
- Fig. 18: N-Acetyl-Asp-Glu-Val-Asp-7-amido-4-trifluoromethylcoumarin;
- Fig. 19: eine schematische Darstellung des G3P-Proteins mit seinen Domänen;
- Fig. 20: schematisch die Absorption des Bakteriophagen M13 an das Bakterium E. coli;
- Fig. 21: die N1-Domäne des g3p-Gens;
- Fig. 22: die N1L1-Domäne des g3p-Gens;
- Fig. 23: die N1 L1 N2-Domäne des g3p-Gens;
- Fig. 24: die N1L1N2L2-Domäne des g3p-Gens;
- Fig. 25: die N1L1N2L2C-Domäne des g3p-Gens;
- Fig. 26: die WT-Domäne des g3p-Gens;
- Fig. 27: ein Agarosegel nach Elektrophorese der Klonierungsprodukte der g3p-Domänen;
- Fig. 28: ein SDS-Gel nach Elektrophorese zur Überprüfung der Expression der G3P-Domänen;
- Fig. 29: ein SDS-Gel nach Elektrophorese zur Überprüfung der gereinigten G3P-Domänen auf ihre Löslichkeit und Reinheit;
- Fig. 30: eine Darstellung einer Linkerregion in einem Fusionsprotein-Gen;
- Fig. 31: Zellkulturen zur Überprüfung der Expression des G3P-N1 L1 N2 und EmGFP als Fusionsprotein;
- Fig. 32: eine Analyse von E.coli nach Expression des Fusionsproteins G3P-N1L1N2 und EmGFP;
- Fig. 33: ein SDS-Gel nach Elektrophorese zur Überprüfung des gereinigten Fusionsproteins G3P-N1 L1 N2 und EmGFP auf seine Löslichkeit und Reinheit;
- Fig. 34: ein Agarosegel nach Elektrophorese zur Überprüfung des gereinigten Fusionsproteins G3P-N1 L1 N2 und Caspase-3 auf seine Löslichkeit und Reinheit;
- Fig. 35: eine schematische Darstellung eines ersten Pull-Down-Experiments mit einem Bakteriophagenprotein zwischen einem Bakterium und einem Metallchelat;
- Fig. 36: eine schematische Darstellung eines zweiten Pull-Down-Experiments ohne ein Bakteriophagenprotein zwischen dem Bakterium und dem Metallchelat;
- Fig. 37: eine graphische Darstellung der Pull-Down-Experimente.

Nachfolgend wird ein Verfahren zur Anreicherung und Detektion von Bakterien 10 durch Bakteriophagenproteine 12 beschrieben.

Die meisten spezifischen mikrobiologischen Detektionsverfahren besitzen gewisse Nachteile, die auf dem zugrundlegenden Verfahren an sich beruhen. Zum Beispiel sind etablierte Nachweismethoden für Mikroorganismen oft sehr zeitintensiv, da die Mikroorganismen zunächst auf Nährmedien überführt und bebrütet werden müssen. Nach dieser Voranreicherung erfolgt dann eine selektive Anreicherungen und anschließend ein biochemisches Screening (API-Test). Oftmals zeigt dieser klassische Nachweisprozess falsch-positive oder falsch-negative Ergebnisse und es müssen immunologische (ELISA) und erbgutbasierende Verfahren (PCR, ISH und FISH) zusätzlich hinzugezogen werden. Diese Verfahren sind in der Routine sehr teuer und zeigen auch in Bezug auf die Identifizierung eine gewisse Unsicherheit. Zum Beispiel zeigt ein ELISA, der auf einem polyklonalen Antikörper 14 aufbaut, oftmals Kreuzreaktionen zu anderen Organismen. Ein weiterer Nachteil dieses immunologischen Detektionsverfahrens ist die Endlichkeit der Ressource, da ein polyklonaler Antikörper durch Immunisierung des Tieres (Hase, Maus, Ziege, Pferd) produziert wird.

Gegenwärtig werden Bakterien 10 durch folgende Methoden detektiert: Kultivierung auf Selektivnährböden, Bunte Reihe (API-Test, VITEK), Polymerase-Kettenreaktion (PCR), Enzyme Linked Immunosorbent Assay (ELISA), Fluoreszenz-in-situ-Hybridisierung (FISH), In-situ-Hybridisierung (ISH), Aptamere.

Es werden nun rekombinant Bakteriophagenproteine 12, die die Adsorption eines Bakteriophagen 16 an eine Bakterienoberfläche 18 ermöglichen, durch Proteinexpression hergestellt. Diese binden spezifisch an der Bakterienoberfläche 18 über Protein-Protein-Interaktionen und können sowohl als Anreicherungsstruktur 19 zur Anreicherung als auch zur Detektion von Bakterien 10 verwendet werden.

Im Folgenden wird Anreicherung von Bakterien 10 durch rekombinante Bakteriophagenproteine 12 beschrieben.

Rekombinant hergestellte Bakteriophagenproteine 12 können an diverse unterschiedliche Materialien immobilisiert bzw. diese Materialien können mit den Bakteriophagenproteinen beschichtet bzw. gecoatet werden. Folgende Materialien können als Oberflächenstruktur 19a beschichtet bzw. gecoatet werden:
1) Magnetische Beads 20, Coating über Biotin 22 - Streptavidin 24 als Biotin-Streptavidin-Linker 25;
2) Magnetische Ni-Beads 26, Coating über Polyhistidin-Tag 28 (His-Tag bzw. 6 x His-Tag) als Metallchelat-Polyhistidin-Tag-Linker 29 über Metallionen wie Nickel, Cobalt oder Kupfer;
3) Nicht magnetische Beads 30, z.B. Ni-NTA 31;
4) Chromatographiesäulen 31 a aller Art der FPLC, Smartsysteme oder HPLC;
5) Sepharosen 31 b wie Cyanogen, bromide-activated-Sepharose;
6) Affinitätssepharosen Glutathione Sepharose;
7) Mikrotiterplatten 31 c;
8) Reaktionsgefäße aller Art aus Kunststoffen 31 d;
9) Filter 31 e für Flüssigkeiten, Gase oder Luft;
10) Spritzen, Schläuche und Ventile;
11) Glas 31f oder Glasgefäße;
12) Kunststoffe 31 d aller Art;
13) Metall 31g oder Metallgefäße,
14) Keramik 31 h oder Keramikgefäße;
15) Holz 31 i, Gestein 31j;
16) Gewebe 31 k, Stoffe;
17) Nanopartikel 31I;
18) Halbleitermaterialien 31m (Si, Si-Nitrit, Gallium-Nitrit).

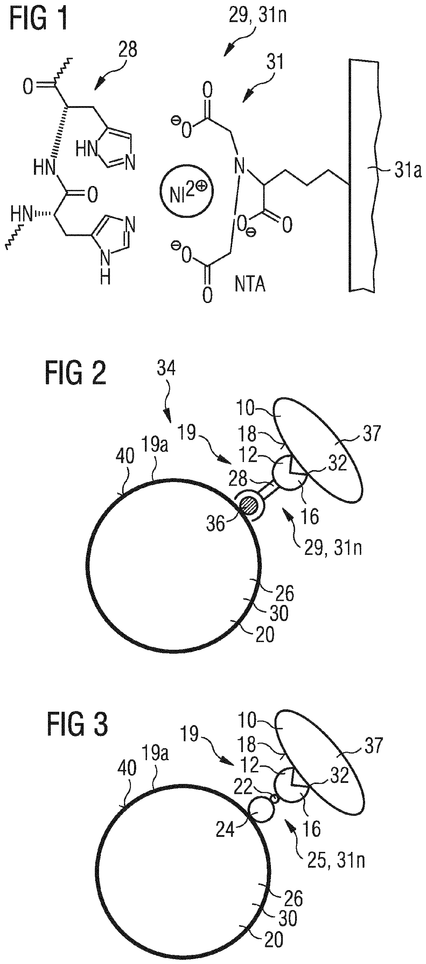

Fig. 1 zeigt Beispiel für ein Coating über Polyhistidine-tag 28 als Linkerstruktur 31 n aus (Quelle: http://www.chemgapedia.de)

Nach der Immobilisierung der Materialien durch die Bakteriophagenproteine 12 können diese zur spezifischen Anreicherung der Bakterien 10 an die oben beschrieben Materialien verwendet werden. Wie schon erwähnt erfolgt die Anreicherung der Bakterien 10 über die spezifische Protein-Protein-Interaktion zwischen Bakteriophagenprotein 12 auf der einen Seite und bakteriellem Oberflächenprotein 32 auf der anderen Seite.

Nachfolgend sind einige Beispiele für eine Anreicherung von Bakterien 10 durch Bakteriophagenproteine 12 beschrieben.

Fig. 2 zeigt eine Anreicherung über magnetische oder nicht magnetische Metallchelat-Beads 34, insbesondere Nickel- oder Cobaltionen, wobei über ein magnetisches oder nicht magnetisches Metallchelat-Bead 34, ein Metallchelat 36, ein Polyhistidine-Tag 28 (His-Tag bzw. 6 x His-Tag) und ein Bakteriophagenprotein 12 ein Bakterium 10 oder nur ein bakterielles Oberflächenprotein 32 einer Bakterienfamilie 37 immobilisiert wird.

Fig. 3 zeigt eine Anreicherung über magnetische 20 oder nichtmagnetische Beads 30 bzw. Sepharosekügelchen 38 durch Biotin 22 - Streptavidin 24 - Interaktion, wobei über magnetische 20 oder nicht magnetische Beads 30 oder Sepharosekügelchen 38, Streptavidin 24, Biotin 22 und ein Bakteriophagenprotein 12 ein Bakterium 10 oder nur ein bakterielles Oberflächenprotein 32 immobilisiert wird.

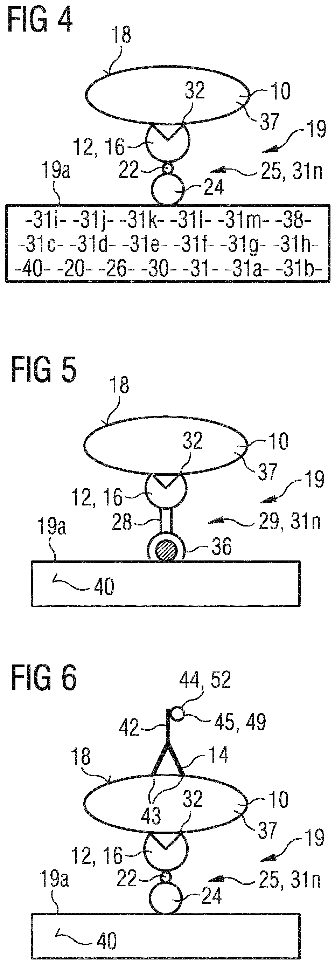

Fig. 4 zeigt eine Immobilisierung der Bakteriophagenproteine 12 über Biotin 22 - Streptavidin 24 an Materialoberfächen 40, insbesondere Mikrotiterplatten 31c, Halbleiter 31 m, Glas 31 f, Metall 31g, wobei an Materialoberflächen 40 aller Art über Streptavidin 24, Biotin 22 und ein Bakteriophagenprotein 12 ein Bakterium10 oder nur ein bakterielles Oberflächenprotein 32 immobilisiert wird.

Fig. 5 zeigt eine Immobilisierung der Bakteriophagenproteine 12 über Metallchelate 36 an Materialoberfächen 40, insbesondere an Mikrotiterplatten 31c, Halbleiter 31 m, Glas 31f oder Metall 31 g, wobei an Materialoberflächen 40 aller Art über ein Metallchelat 36, ein Polyhistidine-Tag 28 (His-Tag bzw. 6 x His-Tag) und ein Bakteriophagenprotein 12 ein Bakterium 10 oder nur ein bakterielles Oberflächenprotein 32 immobilisiert wird.

Nachfolgend wird eine Detektion von Bakterien 10 mit Hilfe von rekombinanten Bakteriophagenproteinen 12 über Protein-Protein-Interaktion erläutert.

Nach dem Binden der Bakterien 10 über Protein-Protein-Interaktion der Bakteriophagenproteine 12 kann eine mögliche Detektion der Keime erfolgen. Nahezu alle für Bakterien 10 bekannten Detektionsverfahren können angewendet werden. Diese Detektionsverfahren sind:
1) Kultivierungsverfahren auf Nährböden und Selektivnährböden (Petrischalen);
2) Kultivierungsverfahren in Flüssigmedien;
3) Biochemische Detektionsverfahren der "Bunten Reihe" (API-Test, VITEK-Test);
4) Detektionsverfahren in Flüssigkeiten mit Indikatoren;
5) Immunoassays, ELISA, Sandwich-ELISA, indirekter ELISA, enzymatisch oder über Fluoreszenz (z.B. HRP oder Fluorescein), Radioimmunoassay (RIA);
6) Sandwich-ELISA oder Hybrid-Phago-Immuno-Assay mit Bakteriophagenprotein-Bakterium-Antikörper, Nachweis enzymatisch oder über Fluoreszenz (z.B. HRP oder Fluorescein);
7) PCR;
8) Real-time PCR;
9) Reverse PCR;
10) Detektion über Aptamere;
11) Hypridisierungsverfahren, FISH;
12) Western-Blot;
13) Southern-Blot;
14) Northern-Blot;
15) Dot-Blot Analyse;
16) Enzymatische Kinetiken;
17) Mikroskopie und mikroskopische Verfahren, Gramfärbung, Methylenblau, Karbolfuchsin, Karbolfuchsin-Methylenblau, CFDA;
18) Elektronenmikroskopie;
19) Ramanverfahren;
20) MALDI-TOF;
21) IMS;
22) GC-MS;
23) MS;
24) GC;
25) Elektronische Nasen, Gassensoren.

Insbesondere können die Bakteriophagenproteine 12 in Kombination mit einem Antikörper 14 eingesetzt werden.

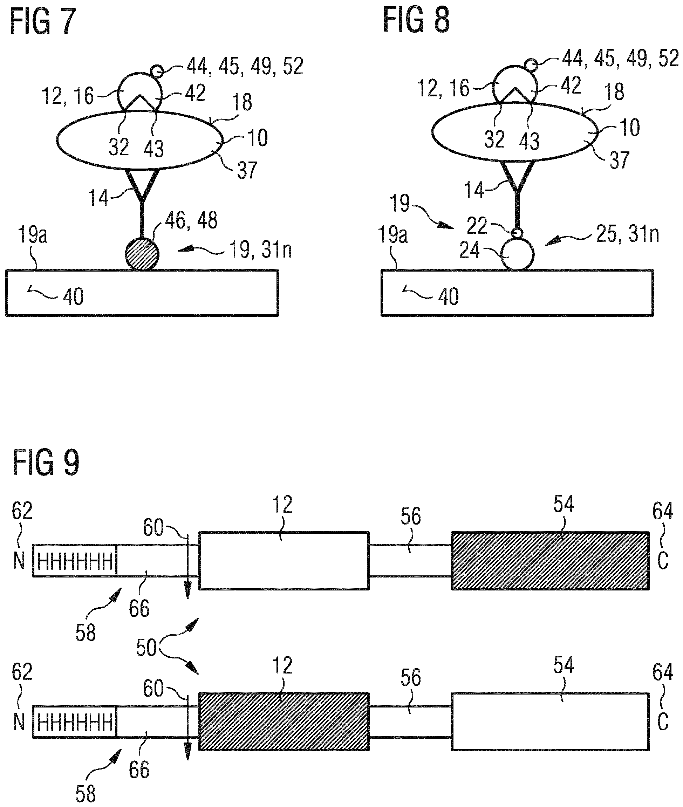

Ein immobilisierter Antikörper 14 könnte als Fänger fungieren, während ein unmittelbar an der Detektionsproteinoberfläche 43 gelabeltes (markiertes) Bakteriophagenprotein 12 als Detektionsprotein 42 zur Anwendung käme, wie beispielsweise in Figs. 7 und 8 gezeigt.

Es handelt sich hierbei nicht um einen klassischen Sandwich-ELISA, der rein aus Antikörpern 14 aufgebaut wurde, sondern vielmehr um ein heterogenes Konstrukt, dessen korrespondierender Assay-Test den Namen Hybrid-Phago-Immuno-Assay (HYPIA) trägt.

Fig. 6 zeigt eine schematische Darstellung einer solchen nicht erfindungsgemäßen immunologischen Detektion über einen markierten Antikörper 14 in Form eines "Hybrid-Phago-Immuno-Assay (HYPIA)" nach einer Anreicherung durch Bakteriphagenproteine 12, wobei an eine Materialoberfläche 40 aller Art über Streptavidin 24, Biotin 22 und ein Bakteriophagenprotein 12 ein Bakterium 10 oder ein bakterielles

Oberflächenprotein 32 immobilisiert wurde und ein monoklonaler oder polyklonaler Antikörper 14, der an das Oberflächenprotein des Bakteriums 10 und eine Markierung 44 bzw. ein Label in Form eines Markermoleküls 45 aufweist, eine Detektion über ein Enzym oder Fluoreszenz (z.B. HRP oder Fluorescein) durchgeführt werden kann.

Figs. 7 und 8 zeigen schematische Darstellungen einer Detektion bzw. eines Assays über markierte (gelabelte) Bakteriophagenproteine 12 nach einer Anreicherung durch Antikörper 14 als "Hybrid-Phago-Immuno-Assay (HYPIA)", wobei an Materialoberflächen 40 aller Art, über Protein A 46 oder Protein G 48 bzw. über Streptavidin 24 und Biotin 22, sowie einen Antikörper 14 ein Bakterium 10 oder nur ein bakterielles Oberflächenprotein 32 immobilisiert wird und dann über ein Bakteriophagenprotein 12 und eine Markierung 44 bzw. Label des Bakteriophagenproteins 12 die Detektion über ein Enzym oder Fluoreszenz (z. B HRP oder Fluorescein) als Fluoreszenzmolekül 49 durchgeführt werden kann.

Nachfolgend wird die Detektion von Bakterien 10 durch rekombinante Bakteriophagenproteine 12 in Form von Fusionsproteinen 50 beschrieben.

Die Rezeptorproteine der Bakteriophagen 16 können nicht nur als sogenannte "Fängerproteine" (Capture-Proteins) zur Anwendung kommen, sondern auch als Detektionsproteine 42. Wie schon oben beschrieben, können die rekombinanten Bakteriophagenproteine 12 nach ihrer Expression und Reinigung mit einem klassischen Detektionsmolekül bzw. Enzym 52 (gelabelt mit HRP, Phosphatase, Peroxidase, Fluorophore wie CFDA) markiert werden. Bei der Labelung bzw. Markierung mit einem Enzym 52 ist bevorzugt, dass das "Labelingprotein" nicht in dem nachzuweisenden Organismus (Bakterium 10) vorkommt. Es könnte sonst zu Kreuzrektionen kommen, die zu falschen Ergebnisse führen könnten.

Eine weitere Möglichkeit des Labelings ist die Herstellung eines Fusionsproteins 50, das aus dem Bakteriophagenprotein 12 auf der einen Seite und dem Labelenzym bzw. Reporterenzym, d.h. einem Markerprotein 54 als Detektorprotein, auf der anderen Seite besteht. Dieses sogenannte Fusionsprotein 50 kann auch als ein rekombinantes Protein in einem Organismus (Expressionsystem) exprimiert werden. Je nach Anwendung kann das Labelingprotein (Detektorprotein 54, Reporterenzym) N-terminal oder C-terminal positioniert werden, wobei vorzugsweise zwischen Fängerprotein und Detektorprotein 54 ein Linker, Linkerprotein 56, von mehreren Aminosäuren eingebaut wird. Der eingebaute Linker, auch Loop genannt, ermöglicht eine Bewegung zwischen Fängerprotein und Detektorprotein 54, so dass dem Fusionsprotein 50 während der Adoption eine gewisse Dynamik zu eigen ist. Zusätzlich besitzt das Fusionsprotein 50 am N-Terminus einen Reinigungs-Tag 58 bzw. Protein- Tags, insbesondere einen Polyhistidin-Tag 28 (His-Tag bzw. 6 x His-Tag), der wiederum C-terminal mit einer Proteaseschnittstelle 60 bzw. Proteaseerkennungssequenz 66 gekoppelt ist. Diese Proteaseschnittstelle 60 ermöglicht es, den His-Tag proteolytisch abzutrennen, so dass nur noch das reine Fusionsprotein 50 bestehend aus Fängerprotein und Detektorprotein 54 übrig bleibt. Folgende Proteaseschnittstellen 60 können zwischen Tag und dem Fusionprotein 50 eingebaut werden:
1) Thorombin;
2) TEV-Protease;
3) Faktor XA;
4) 3C Protease (PreScission Protease);
5) Enterokinase.

Auch die Verwendung unterschiedlicher Reinigungs-Tags 58 ist möglich. Der N-terminale Tag kann für gewöhnlich zwei Funktionen wahrnehmen. Diese wären zum einen die nach der Expression anschließende Reinigung über den Tag mit Affinitätschromatographie und zum anderen kann der Tag sich günstig auf die Faltung des Proteins auswirken. Folgende Tags können zum Einsatz kommen:
1) 18A-Tag;
2) ACP-Tag;
3) Avi-Tag;
4) BCCP-Tag;
5) c-myc-Tag;
6) Calmodulin-Tag (CaM-Tag);
7) Chitin-bindendes-Protein-Tag (CBP-Tag);
8) ELK16-Tag;
9) ELP-Tag;
10) FLAG-Tag;
11) Flash-Tag;
12) Glutathion-S-Transferase-Tag (GST-Tag);
13) Hämagglutinin-Tag (HA-Tag);
14) poly-Histidin-Tag (His-Tag bzw. 6 x His-Tag);
15) Isopep Tag;
16) Maltosebinding protein-Tag (MBP-Tag);
17) Nus-Tag;
18) ProtA-Tag;
19) ProtC-Tag;
20) S-Tag;
21) SBP-Tag;
22) Snap-Tag;
23) SpyTag;
24) SofTag 1 und 3;
25) Streptavidin-Tag (Strep-Tag);
26) Strep-II-Tag;
27) Tandem-Affinity-Purification-Tag (TAP-Tag);
28) TC-Tag;
29) Thioredoxin-Tag (TRX-Tag);
30) Ty-Tag;
31) V5-Tag;
32) Xpress-Tag.

Fig. 9 zeigt schematisch Darstellungen eines Fusionsproteins 50, bestehend aus Bakteriophagenprotein 12 (Fängerprotein, Captureprotein) und einem Labelingprotein bzw. Detektorprotein 54, mit der folgenden Abfolge vom N-Terminus 62 zum C-Terminus 64: Proetin-Tag, insbesondere ein poly-Histidin-Tag (His-Tag bzw. 6 x His-Tag), Proteaseerkennungssequenz 66, Proteaseschnittstelle 60, Bakteriophagenprotein 12 (Fängerprotein oder Captureprotein), Linker 56 oder Loop, Labelingprotein bzw. Detektorprotein 54.

In Bezug auf das Bakteriophagenprotein 12 (Fängerprotein, Captureprotein) kann jedwedes bekannte Adsorptionsprotein eines Bakteriophagen 16 verwendet werden. Gerade durch Auswahl eines Bakteriophagenproteins 12 kann entweder ein Bakterium 10 extrem selektiv detektiert werden (z.B. E. coli) oder es besteht die Möglichkeit, dass eine ganze Bakterienfamilie 37 (z.B. Coliforme) sensitiv erfasst wird. Ein bekanntes Beispiel ist das G3P-Protein des Bakteriophagen M13, das an das tolA-Protein 84 von E. coli bindet, wie weiter unten ausführlich beschrieben wird.

Die folgende Tabelle zeigt das G3P-Protein mit seinen nahverwandten Proteinen, die alle als Fängerprotein bzw. Captureprotein verwendet werden könnten.

| Assessions Nr. | Proteinname | Organismus | Aminosäurenlänge |
|---|---|---|---|
| P69169 | Attachment protein G3P (Gene 3 protein) (G3P) (Minor coat protein) | Enterobacteria phage f1 (Bacteriophage f1) | 424 |
| P03661 | Attachment protein G3P (Gene 3 protein) (G3P) (Minor coat protein) | Enterobacteria phage fd (Bacteriophage fd) | 424 |
| P15415 | Attachment protein G3P (Gene 3 protein) (G3P) (Minor coat protein) | Enterobacteria phage 12-2 (Bacteriophage 12-2) | 434 |
| 080297 | Attachment protein G3P (Gene 3 protein) (G3P) (Minor coat protein) | Enterobacteria phage If1 (Bacteriophage If1) | 460 |
| P03663 | Attachment protein G3P (Gene 3 protein) (G3P) (Minor coat protein) | Enterobacteria phage Ike (Bacteriophage IKe) | 434 |
| P69168 | Attachment protein G3P (Gene 3 protein) (G3P) (Minor coat protein) | Enterobacteria phage M13 (Bacteriophage M13) | 424 |
| P25129 | Attachment protein G3P (Gene 3 protein) (G3P) (Minor coat protein) | Pseudomonas phage Pf1 (Bacteriophage Pf1) | 437 |
| P03624 | Attachment protein G3P (Gene 3 protein) (G3P) (Minor coat protein) | Pseudomonas phage Pf3 (Bacteriophage Pf3) | 483 |
| 037972 | Attachment protein G3P (Gene 3 protein) (G3P) (Minor coatprotein) | Xanthomonas phage phiLf (Bacteriophage phi-Lf) | 367 |

Als Labelingprotein oder Detektorprotein 54 können die unterschiedlichsten Proteine 67 sowie alle Enzyme 52, bei denen ein Enzym-Assay etablierte wurde, zur Anwendung kommen. Bevorzugt ist, wie schon bei dem herkömmlichen Labeling beschrieben, dass das Enzym 52 in dem zu detektierenden Organismus nicht existent ist. Auch kein nah verwandtes Enzym 52 sollte sich vorzugsweise in dem Organismus befinden, da man sonst Kreuzreaktionen erwarten würde, was dann zu falsch-positiven Ergebnissen führen würde. Besonders geeignet sind alle Enzyme 52, die nicht in Bakterien 10 existieren und vorzugsweise nur in phylogenetisch weit entfernten Eukaryoten vorkommen. Insbesondere folgende Detektorproteine 54 eignen sich für die Herstellung eines Fusionsproteins 50:
1) Alle fluoreszierenden Proteine 72, insbesondere das grün fluoreszierende Protein (GFP) mit seinen nahen Verwandten CFP, YFP, RFP, EmGFP und BFP;
2) Alle bekannten humanen Caspasen 68 (CASP1, CASP2, CASP8, CASP9, CASP10, CASP3, CASP6, CASP7) insbesondere die Caspase-3 70. Mit der Verwendung der Caspase-3 70 stehen mehrere unterschiedliche Detektionssubstrate zur Verfügung, so dass eine Detektion über Kolorimetrie, Fluoreszenz oder Biolumineszenz möglich ist;
3) Alle auf Biolumineszenz beruhenden Enzyme 52, insbesondere Luziferase 71, z.B. die Firefly- Luziferase;
4) Alle Polymerasen (DNA und RNA), insbesondere Tag-Polymerase 75. Hier sei zu erwähnen, dass mit der Verwendung der Tag-Polymerase 75 vorteilhaft zwei voneinander unabhängige Detektionverfahren, Absorption des Bakteriophagenproteins 12 und PCR, miteinander gekoppelt wurden. Diese Kopplung bietet eine doppelte Sicherheit in Bezug auf falsch-positive Ergebnisse.

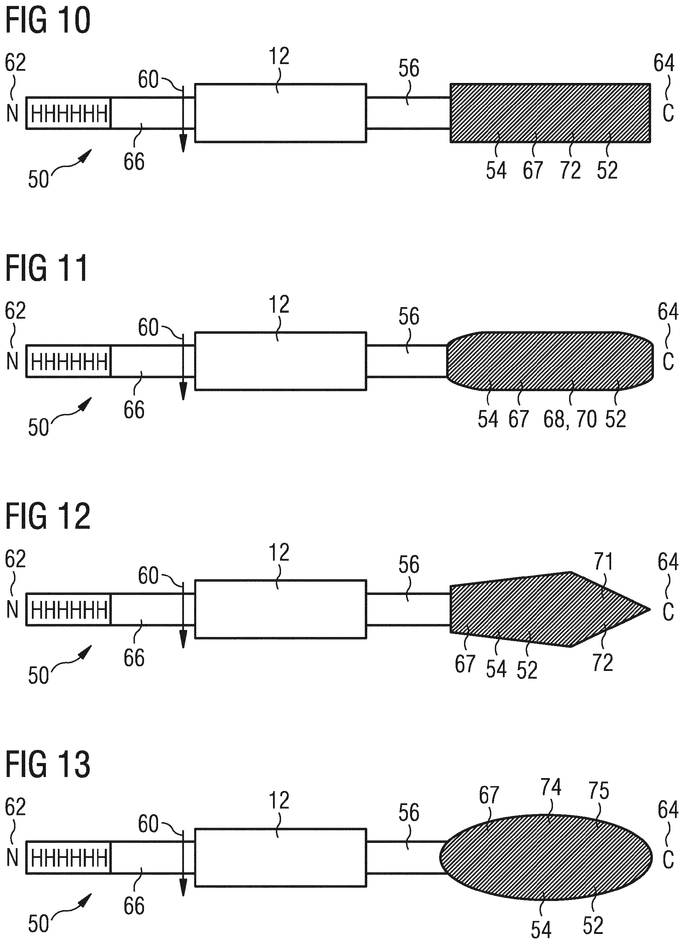

Figs. 10 bis 13 zeigen schematische Darstellungen von Fusionsproteinen 50 mit unterschiedlichen Detektorproteinen 54, nämlich in Fig. 10 ein fluoreszierendes Protein 72, insbesondere das grün fluoreszierende Protein (GFP), in Fig. 11 eine humane Caspase 68, insbesondere die Caspase-3 70, in Fig. 12 ein auf Biolumineszenz beruhendes Enzym 52, insbesondere Luziferase 71, in Fig. 13 eine Polymerase (DNA und RNA) 74, insbesondere Taq-Polymerase 75.

Auch mit den in den Figs. 10 bis 13 schematisch dargestellten Fusionsproteinen 50 lassen sich sehr gut unterschiedlichste Assays aufbauen. Die Assays gleichen in dem Aufbau den Tests mit den herkömmlich markierten (gelabelten) Bakteriophagenproteinen 12 in Figs. 7 und 8.

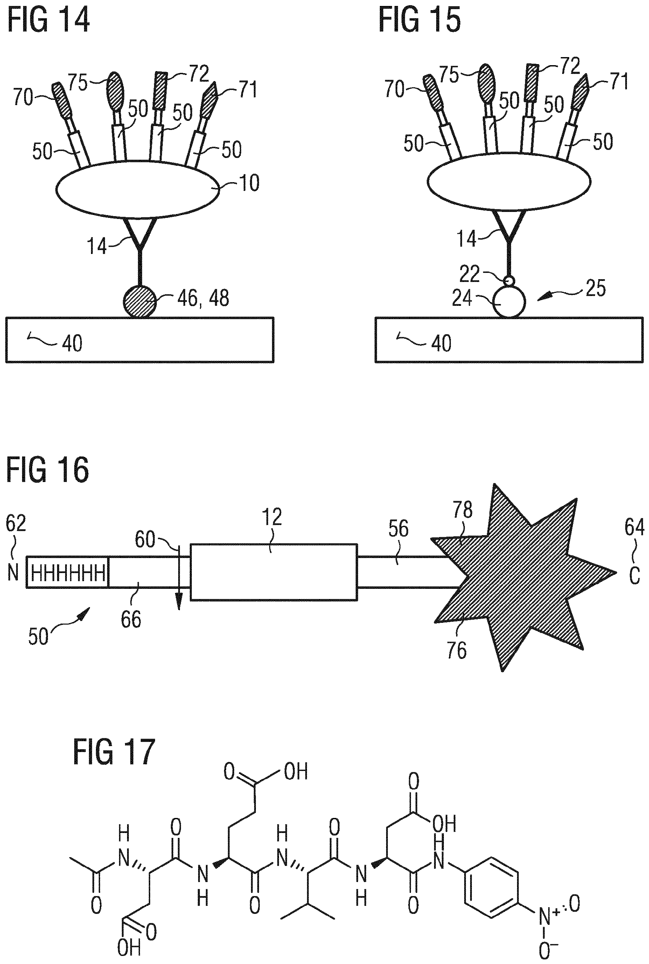

Figs. 14 und 15 zeigen schematische Darstellungen einer Detektion bzw. eines Assays mit einem Fusionsprotein 50 (Bakteriophagenprotein 12 und Detektorprotein 54) nach einer Anreicherung durch Antikörper 14, wobei an einer Materialoberfläche 40 aller Art über Protein A 46 oder Protein G 48 und einen Antikörper 14 ein Bakterium 10 oder nur ein bakterielles Oberflächenprotein 32 immobilisert wurde. An die gegenüberliegende Seite des Bakteriums 10 sind verschiedene Fusionsproteine 50 angelagert, aufweisend Markerproteine 54 in Form von humanen Caspasen 68, insbesondere Caspase-3 70, Polymerasen 74 (DNA und RNA), insbesondere Tag- Polymerase 75, fluoreszierendes Proteine 72, insbesondere das grün fluoreszierende Protein (GFP) und auf Biolumineszenz beruhende Enzyme 52, insbesondere Luziferase 71.

Nachfolgend wird die Anwendung der Bakteriophagenproteine 12 als Fusionproteine 50 in Kombination mit lytischen Proteinen 76 beschrieben.

Zur Erweiterung des Anwendungsbereiches wäre es auch denkbar, das absorbierende Bakteriophagenprotein 12 mit einem lytischen Bakteriophagenprotein 78 oder allgemein lytischen Poteinen 76 (z.B. Lysostaphin, Lysozym) zu fusionieren und das gesamte Konstrukt als Fusionsprotein 50 zu exprimieren. Folgende Anwendungen wären denkbar:
1) Desinfektion, Dekontamination;
2) therapeutische Anwendungen ähnlich der Bakteriophagen 16-Therapie;
3) Antibiotikaersatz, insbesondere gegen multiresistente Keime (MRSA, Tuberkulose);
4) prophylaktische Anwendung in der Lebensmittelindustrie;
5) in der Landwirtschaft bei der Bekämpfung bakterieller Krankheiten (z.B. Feuerbrand bei Pflanzen oder Faulbrut in der Bienenzucht)

Fig. 16 zeigt eine schematische Darstellung eines Fusionsproteins 50, bestehend aus einem Bakteriophagenprotein 12 (Fängerprotein, Captureprotein) und einem lytischen Protein 76 (z. B. Lysozym oder Lysostaphin) sowie einem Protein-Tag, insbesondere ein poly- Histidin-Tag (His-Tag bzw. 6 x His-Tag), einer Proteaseerkennungssequenz 66, einer Proteaseschnittstelle 60, einem Linker 56 oder Loop, und einem lytischen Protein 76.

Die folgenden Expressionssysteme können verwendet werden:
Bakteriophagenproteine 12 oder auch die Fusionsproteine 50 können in prokaryotischen als auch eukarytischen Organismen 80 als Expressionssysteme rekombinat hergestellt werden. Beispiele sind:
   Prokaryotisch:
      1) Escherichia coli für rekombinante Proteine, pET Expression System;
      2) Bacillus subtilis.
   Eukaryotisch:
      1) Hefe (Saccharomyces cerevisiae, Pichia pastoris);
      2) Pilze hauptsächlich für Sekundärmetaboliten (z.B. Antibiotika);
      3) Säugetierzellen (CHO, Myelomzellen);
      4) Insektenzellen (Sf-9, Sf-21) mit Baculovirus-Expressionssystem;
      5) transgene Tiere (z. B. Expression der Milch mit speziellem Casein-Promotor, Mäuse);
      6) transgene Pflanzen (z. B. Mais, Tabak);
      7) Frosch-Oocyten.

Der Aufschluss und die Reinigung der Bakteriophagenproteine 12 wird nachfolgend erläutert.

Nach der Expression werden die Zellen 81 aufgeschlossen und bevorzugt über Chromatographie gereinigt. Folgende Methoden des Zellaufschlusses kommen zur Anwendung:
1) Ultraschall (BRANSON SONIFIER);
2) Aufschluss mit Glasbeads (Ribolyser, BeadBeater);
3) Aufschluss mit flüssigem Stickstoff und Zermahlen, z.B. mit Mörser und Pistille, oder mit einer Kugelmühle;
4) French Press;
5) Einfrieren bei -20°C und Auftauen;
6) Boilingmethode, Aufschluss bei 100°C bevorzugt bei thermostabilen Proteinen;
7) Homogenisator;
8) Biochemischer Aufschluss durch lytische Enzyme, z.B. Lysozym oder Lysostaphin.

Der nächste Schritt zur Aufarbeitung des rekombinanten Proteins ist das Zentrifugieren der aufgeschlossen Zellen 81 zu einem Proteinrohextrakt 81 a. Dies kann unter Verwendung unterschiedlicher Zentrifugationstechniken und G-Zahlen erfolgen. Insbesondere wird der Rohextrakt 81 a für 30 min bei 4 °C zwischen 20.000 G und 50.000 G zentrifugiert. Auch ein Ultrazentrifugationsschritt für 1 h - 3 h bei 4 °C in den Bereichen 100.000 g bis 150.000G kann der ersten Zentrifugation folgen. Nach der Bereitung des Proteinrohextraktes erfolgt die Proteinreinigung mittels Chromatographie (Fast Protein Liquid Chromatography: FPLC). Als Proteinreinungsmethoden können folgende Techniken zu Anwendung kommen:
Chromatographie:
   1) lonenaustauschchromatographie (IEX);
   2) Größenausschlusschromatographie (SEC);
   3) Affinitätschromatographie;
   4) polare Chromatographie;
   5) Hydrophobe Interaktionschromatographie (HIC);
   6) Umkehrphasen-Chromatographie (RPC).
Extraktion, Fällung und Filtration:
   1) serielle Extraktionen;
   2) serielle Fällungen (z.B. Ammoniumsulfat);
   3) Filtration.

Mehrere Verbesserungen und Vorteile ergeben sich durch dieses Vorgehen:
Auf Grund der rekombinanten Herstellung der Bakteriophagenproteine 12 bzw. Fusionsproteine 50 entsteht eine immer gleichbleibende Qualität der Produkte, die sich in dem gleichbleibenden Bindungkoeffizienten der Bakteriophagenproteine 12 an die bakteriellen Oberflächenproteine 32 wiederspiegelt. Im Vergleich zu einem polyklonalen Antikörper 14, der erhebliche Schwankungen in Bezug auf Kreuzreaktionen aufweist, sind diese bei einem rekombinant hergestellten Bakteriophagenprotein 12 nahezu ausgeschlossen. Grund hierfür sind die zwei völlig unterschiedlichen Herstellungsverfahren. Bei der Herstellung des polyklonalen Antikörpers 14 wird in der Regel ein Säugetier (Hase, Maus, Ziege) immunisiert. Mit anderen Worten es wir dem Tier ein Antigen (Protein, Peptid) gespritzt, gegen das dann das Tier den Antikörper 14 bildet. Hingegen basiert die Herstellung von rekombinanten Proteinen auf der genetischen bzw. gentechnolgischen Expression des Proteins in Kulturen niederer Organismen 80 (siehe Herstellung von Impfstoffen oder Biopharmazeutik).

Außerdem ist die Immunisierung eines Hasen oder einer Ziege ein äußerst kostenaufwändiges Verfahren, wohingegen die Herstellung eines rekombinanten Proteins relativ kostengünstig ist. Auch ist die Verwendung monoklonaler Antikörper 14, die ohne Immunisierung eines Tieres hergestellt werden, ein äußerst aufwendiges und kostspieliges Verfahren.

Auch stellt ein polyklonaler Antikörper 14 eine endliche Ressource dar, da das immunisierte Tier irgendwann stirbt, während die Herstellung rekombinanter Proteine eine endlose Ressource ist.

Durch Verwendung eines Fermenters bzw. Bioreaktors lassen sich große Mengen des rekombinaten Proteins herstellen (Gramm- bis Kilogrammbereich). Dies ist bei der Herstellung eines polyklonalen oder monoklonalen Antikörpers 14 nicht möglich.

Auch der ethische und moralische Aspekt sei zu berücksichtigen. Bei der Herstellung von polyklonalen Antikörpern 14 werden zur Immunisierung meistens höhere Organismen (meistens Säugetiere) verwendet. Die Herstellung der rekombinanten Proteine erfolgt meisten in Bakterien 10 oder niederen Organismen 80 (z. B. Hefen).

Auf Grund der hoch spezifischen Protein-Protein-Interaktion zwischen Bakteriophagenprotein 12 und bakteriellem Oberflächenprotein 32 entstehen keine oder kaum Kreuzreaktionen und die Bindung weist eine extrem hohe Affinität auf.

Ein äußerst großer Vorteil bei dem vorgestellten Hybrid-Phago-Immuno-Assay (HYPIA) (vgl. Figs. 6 bis 8) ist das Binden der beiden Adsorptionsproteine (Antikörper 14 und Bakteriophagenprotein 12) an unterschiedliche Epitope, wodurch die Sensitivität und Sicherheit das Assays erhöht wird.

Bei der Reinigung des rekombinaten His-Tag-Proteins entsteht ein Vorteil in Bezug auf die Assayentwicklung. Da das rekombinante Protein schon an den Beads (Ni-NTA 31, oder magnetische Ni- Beads 26) über den His-Tag gebunden ist, können diese umgehend zur Anreicherung oder Assayentwicklung verwendet werden. Es ist also nicht mehr notwendig, das rekombinante Protein von den Beads zu eluieren. Auch mit Nickelionen beschichtete Mikrotiterplatten 31 c oder andere Gefäße können zur Reinigung verwendet werden.

Bei der rekombinanten Herstellung von Fusionsproteinen 50 (Bakteriophagenprotein 12 und Detektorprotein 54, z.B. GFP) wird nur ein Label pro Bakteriophagenprotein 12 angehängt, wohingegen bei dem herkömmlichen Labeling eine große Schwankungsbreite in Bezug auf das Coating des Proteins zu erwarten ist. Häufig werden mehre Enzyme 52 (z.B. HRP) bzw. Fluorophoren gebunden, was eine große Auswirkung in Bezug auf die Sensitivität des Assays hat. Auch ist das herkömmliche Labeling kein 100%iges Markieren. Häufig wird nur ein Bruchteil des zu labelnden Proteins markiert (manchmal nur 10 %). Dieses unvollständige Markieren kann bei der Herstellung rekombinanter Fusionsproteine 50 nahezu ausgeschlossen werden (nur ein Bakteriophagenprotein 12 und ein Reporterprotein).

Die Verwendung der humanen Caspase-3 70 als Reporterenzym besitzt sehr große Vorteile, da es sich um ein rein humanes Enzym 52 handelt und nicht in den nachzuweisenden Bakterien 10 vorkommt Die enzymatische Reaktion beruht auf einer proteolytischen Spaltung mit folgender Aminosäureerkennungssequenz: DEVDIX. Diese Erkennungssequenz findet sich äußerst selten in bakteriellen Proteinen, so dass deren proteolytische Spaltung so gut wie ausgeschlossen ist.

Folgende Substrate werden bei der Caspase-3 70 für den Aufbau eines Assays verwendet:
Fig. 17 zeigt ein kolorimetrisches Substrat, das N-Acetyl-Asp-Glu-Val-Asp p-nitroanilid.

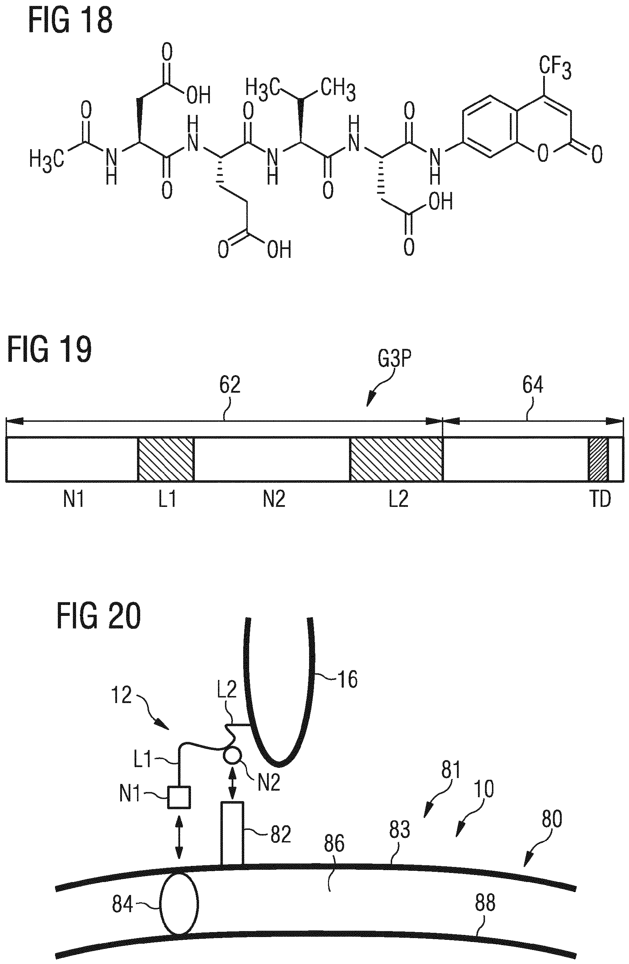

Fig. 18 zeigt ein Fluoreszenzsubstrat, das N-Acetyl-Asp-Glu-Val-Asp-7-amido-4-trifluoromethylcoumarin.

Weitere mögliche Fluoreszenzsubstrate sind N-Acetyl-Asp-Glu-Vai-Asp-7-amido-4-methylcoumarin und N-Acetyl-Asp-Gln-Met-Asp-7-amido-4-trifluoromethylcoumarin.

Bei der Verwendung der Tag-Polymerase 75 als Reporterenzym in Form eines Fusionsproteins 50 mit einem Bakteriophagenprotein 12 als "Capture-Protein" ergibt sich der Vorteil der doppelten Spezifität. Zum einen ist die Protein-Protein-Interaktion zwischen Bakteriophagenprotein 12 und bakteriellem Oberflächenprotein 32 hoch spezifisch und zum anderen ist der nachfolgende Detektions-Assay, in dem Fall eine spezifische PCR, ebenfalls ein hoch sensitiver und spezifischer Nachweis. Diese doppelte Spezifität bei der Identifizierung erhöht die Sicherheit des Assays, so dass falsch-positive Ergebnisse so gut wie ausgeschlossen sind. Auch in einer Real-Time-PCR kann dieses Fusionsprotein 50 eingesetzt werden.

In vielen Fällen ist es wünschenswert, die rekombinanten Proteine vor einem Abbau zu schützen. Durch Maßnahmen, die den Abbau verhindern bzw. verzögern, könnten erhebliche Vorteile in Bezug auf die Lagerung gegenüber herkömmlichen immunogischen oder enzymatischen Tests generiert werden. Folgende Maßnahmen können die Proteine gegen Abbau schützen:
1) Stabilisierung mit dem Vakuumtrocknungsverfahren;
2) Stabilisierung durch Gefriertrocknung bzw. Lyophilisation oder Sublimationstrocknung;
3) Stabilisierung durch Zugabe von Sacchariden, Sucrose, Glycerol, Polyolen, Polyethylenglykol, Aminosäuren, Methylamine und/oder anorganischen Salzen;
4) Stabilisierung durch PEGylierung; hier wäre auch eine therapeutische Anwendung möglich, denn das Verfahren des PEGylierens kommt häufig bei Biopharmazeutika zum Einsatz, die intravenös gespritzt werden.

Auch ist das Bakteriophagenprotein 12 ohnehin ein wesentlich stabileres Makromolekül als ein Antikörper 14, der aus mehreren Fragmenten (leichte und schwere Kette) aufgebaut ist. Weiterhin besteht die Möglichkeit, das Bakteriophagenprotein 12 durch "Protein-Engineering" gezielt zu modifizieren, sodass die Stabilität des Bakteriophagenproteins 12 erheblich gesteigert werden kann.

Nachfolgend wird ein experimentelles Beispiel für die Herstellung und Verwendung von Bakteriophagenproteinen 12 zur Anreicherung und Detektion beschrieben.

Zunächst wird kurz die Adsorption von Bakteriophagen 16 an ihre Wirtsbakterien am Beispiel des Bakteriophagen M13 an E. coli erläutert.

Das hier vorgestellte neuartige Anreicherungs- und Detektionverfahren beruht auf der Verwendung von rekombinierten Bakteriophagenproteinen 12, die über Protein-Protein-Interaktionen an der Bakterienoberfläche 18 binden. Bakteriophagen 16 sind Viren, die Bakterien 10 befallen und diese als Wirtszellen benutzen. Die Vermehrung der Bakteriophagen 16 verläuft oftmals in Form eines lydischen Zykluses, der mit der Adsorption des Bakteriophagen 16 an der die Bakterienoberfläche 18 beginnt. Es erfolgt dann die eigentliche Reproduktion des Bakteriophagen 16, die in mehreren Schritten abläuft:
1) Injektion des Bakteriophagenerbguts (DNA) in die Wirtszelle;
2) Latenzphase, Replikation des Bakteriophagenerbguts;
3) Produktionsphase, Produktion von Bakteriophagenbestandteilen;
4) Reifephase, Assembly zum vollständigen Bakteriophagen 16;
5) Lyse bzw. Freisetzung der fertigen Bakteriophagen 16 und erneute Infektion.

Der hier zugrunde liegen Schritt ist die Adsorption des Bakteriophagens 16 an der Oberflächenstruktur des Bakteriums 10. Diese Adsorption erfolgt über eine äußerst spezifische Protein- Protein-Interaktion zwischen Virus und Wirtszelle. Als praktisches Beispiel dient hier der Bakteriophage M13 und das Bakterium E. coli.

Die Absorption des Bakteriophagen 16 erfolgt über das Gen-3-Protein (g3p, SEQ ID NO 1, SEQ ID NO 2), das auf der Bakterienseite an das tolA-Protein 84 bindet. Das g3p-Gen besitzt eine Länge von 1275 Basenpaaren (bp) und codiert ein Protein mit der Länge von 424 Aminosäuren (AS). Auf Proteinebene besteht das G3P im N-terminalen Bereich aus vier Domänen, die, wie in Fig. 19 dargestellt, jeweils mit N1, L1, N2 und L2 bezeichnet werden, während der C-terminale Abschnitt mit einer kurzen Transmembrandomäne TD gekennzeichnet ist. Die Interaktion des Bakteriophagen 16 ist in der Literatur gut beschrieben und erfolgt zunächst mit der N2-Domäne, die an den F-Pilus 82 von E. coli bindet, der an der äußeren Membran 83 lokalisiert ist. Im nächsten Schritt interagiert dann die N1-Domäne des Bakteriophagen 16 mit dem bakteriellen tolA-Protein 84, das sich in dem periplasmatischen Raum 86 zwischen der äußeren Membran 83 und der Cytoplasmamembran 88 befindet. Nach der Absorption über die beiden Domänen erfolgt dann die eigentliche Infektion des Bakteriums 10 durch den Bakteriophagen 16, indem das Virus sein Erbgut (DNA) in die Wirtszelle injiziert.

Fig. 19 zeigt schematisch das G3P-Protein mit seinen N-teminalen Domänen N1, L1, N2 und L2. C-terminal lokalisiert befindet sich die Transmembrandomäne TD.

Fig. 20 zeigt schematisch eine Darstellung der Absorption des Bakteriophagen M13 an das Bakterium E. coli.

Die nachfolgende Tabelle beschreibt die Lokalisierung der g3p-Domänen aus Protein- und Genebene:

| Domäne | Lokalisierung im Protein | Lokalisierung im Gen |
|---|---|---|
| N1 | 1-85 AS | 1-255 bp |
| L1 | 86-104 AS | 255-315 bp |
| N2 | 104-235 AS | 315-705 bp |
| L2 | 235-274 AS | 705-822 bp |
| TD | 398-418 AS | 1191-1257bp |
| N-Terminus | 1-274 AS | 1-822 bp |
| C-Terminus | 274-424 AS | 822-1275 bp |
| g3p (volle Länge) | 1-424 AS | 1-1275 bp |

Nachfolgend wird die Klonierungstrategie von g3p-Konstrukten in einen T7-basierenden Expressionsvektor beschrieben.

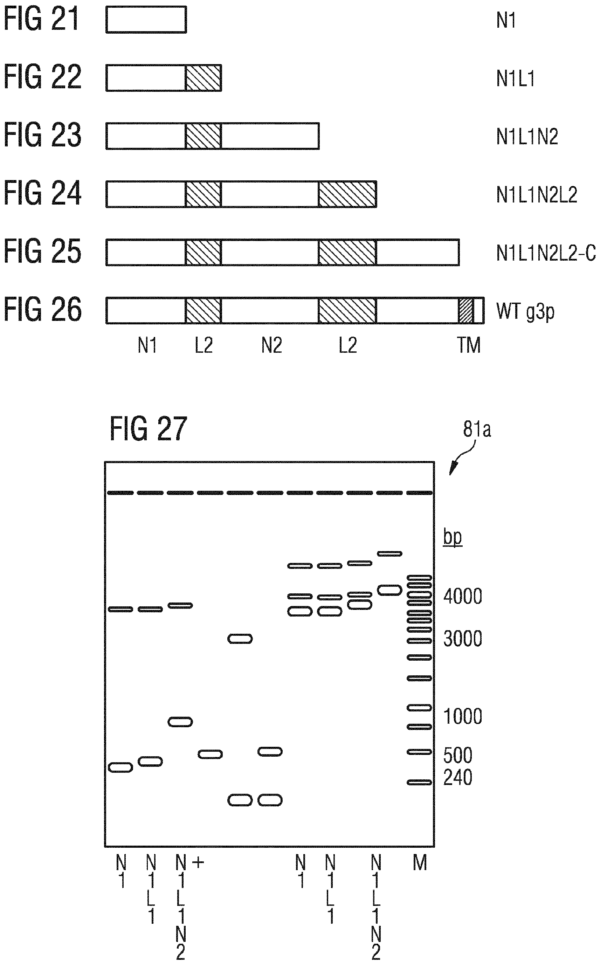

Zur Herstellung von rekombiniertem G3P-Protein wurde das Volllängenprotein zunächst in fünf unterschiedliche Genfragmente eingeteilt. Die Einteilung der Genfragmente erfolgte nach den oben beschrieben Domänen (N1, L1, N2, L2), wobei das kleinste Fragment nur die N1-Domäne und das längste Fragment alle Domänen bis hin zur Transmembrandomäne (TD) beinhaltete. Zunächst wurden g3p-spezifische Oligonukleotide bzw. Primer designt, die eine Amplifizierung dieser Fragmente mittels PCR ermöglichten. Für die spätere Klonierung der PCR-Produkte in ein T7-basierendes Expressionssystem wurde jeweils an den 5'-Enden der "forward (F)"- und "reverse (R)"-Primer eine Restriktions-schnittstelle mit sechs zusätzlichen Nukleotiden (ACGTAC) angehängt. Diese zusätzlichen Nukleotide dienten der optimalen Interaktion und Katalyse der Restriktionsendonuklease. Als Restriktionsendonukleasen wurde der F-Primer mit einer BsmBI-Schnittstelle und alle R-Primer mit einer Acc651-Schnittstelle ausgestattet. Normalerweise wird der F- Primer mit einer Ncol-Schnittstelle designt, da das Palindrom von Ncol (5'-...CvCATGG-...3') das klassische Startcodon ATG beinhaltet. Für die Verwendung von Ncol ist es aber bevorzugt, dass die erste Base nach dem Startcodon ein G ist. Dies ist bei dem g3p-Gen jedoch nicht der Fall und es muss auf eine andere Klonierungsstrategie ausgewichen werden. Durch Verwendung des Restriktionenzyms 90 BsmBI ist es jedoch möglich, ein "Sticky end" zu generieren, das dem eines Ncol-Schnitt gleichkommt. Das Palindrom von BsmBI zeigt die Sequenz 5'-...CGTCTC(N)1 v...-3', die in einem Musterprimer wie folgt aussehen würde: 5'-ACGTACCGTCTCCvCATG....3'. Nach dem Restriktionverdau ist es daher möglich, das BsmBI-PCR-Produkt mit einem "Sticky end", das von Ncol generiert wurde (z.B. der mit Ncol verdaute Expresionsvektor), zu ligieren. Insgesamt wurden folgende fünf PCR-Produkte bzw. "Inserts" mit Hilfe einer "Proof-Reading-DNA-Polymerase" amplifiziert:
1) N1 (255bp), Fig. 21;
2) N1L1 (315bp). Fig. 22;
3) N1L1N2 (705bp), Fig. 23;
4) N1 L1 N2L2 (822bp), Fig. 24;
5) N1 L1 N2L2-C (C:C-Terminus, Fragment bis zur TM, 1191bp), Fig. 25.

Nach der Amplifikation wurden die PCR-Produkte seriell mit den Restriktionsenzymen Acc651 und BsmBI verdaut und in einen T7-basierenden Expresionsvektor ligiert, der zuvor mit den Restriktionsenzymen Ncol und Acc65I geöffnet wurde, wie in Fig. 26 gezeigt. Die Transformation in E. coli NEB 5-alpha erfolgte mit Elektroporation, wobei die Ligationen durch eine Butanolfällung salzfrei gemacht wurden. Es erfolgte eine Selektion auf LB-Kanamycin-Nährböden (Endkonzentration von etwa 50 µg/ml Kanamycin). Im nachfolgenden wurde von den Transformanden eine DNA-Mini-Plasmid-Präparation durchgeführt, wobei anschließend die präparierte Plasmid-DNA durch einen Restriktionsverdau sowie durch PCR auf ihre Richtigkeit in Bezug auf die g3p-Fagmente überprüft wurde.

Die nachfolgende Tabelle zeigt die verwendeten Primer bzw. Oligonukleotide mit ihren Restriktionsschnittstellen, die für die Amplifizierung der fünf unterschiedlichen Genfragmente verwendet wurden:

| Fragment | Richtung | Name | Sequenz |
|---|---|---|---|
| alle Fragmente | F | g3p/Bsmbl/fw | |
| N1 | R | N1/S/Ac/rv | |
| N1L1 | R | N1L1/S/Ac/rv | |
| N1L1N2 | R | N1L1N2/S/Ac/rv | |
| N1L2N2L2 | R | N1L1N2L2/S/Ac/rv | |
| N1L2N2L2-C | R | g3p/oT/S/Ac/rv | |

Fig. 27 zeigt das Ergebnis einer Elektrophorese eines Agarosegels, das die PCR-Produkte sowie die Klonierungsprodukte der g3p-Fragmente N1, N1L1 sowie N1 L2N2 zeigt. Die ersten drei Spalten von links zeigen die PCR-Produkte N1 (255bp), N1L1 (315bp) und N1 L2N2 (705bp), die vierte Spalte von links die Positivkontrolle, die siebte bis neunte Spalte von links die Legierung der PCR-Produkte N1, N1L1 sowie N1 L2N2 mit dem T7-basierenden Expressionsvektor und die elfte Spalte von links den DNA-Marker M.

Nachfolgend wird die Expression der G3P-Domänen N1, N1L1 und N1 L1 N2 als rekombinantes 6 x His-Tag-Protein erläutert.

Die fertigen Konstrukte (g3p-Domänen in T7-Expressionsvektor) wurden in kalziumkompetente E. coli BL21 (DE3) transformiert und anschließend auf LB-Kanamycin-Nährböden (Endkonzentration von etwa 50 µg/ml Medium) selektiert. Zur eigentlichen Proteinexpression wurden mehr Kolonien der Transformanten von den Selektivplatten entnommen und es wurde mit diesen eine Vorkultur von 5 - 10 ml LB und Kanamycin 50 µg/ml inokuliert. Nach einer Inkubation im Schüttler (200 rpm) über Nacht bei 37 °C wurde die Hauptkultur (100 ml - 1000 ml LB und Kanamycin 50 pg/ml) mit der Vorkultur beimpft. Nach der Beimpfung sollte die Hauptkultur eine optische Dichte bei 600 nm (OD 600) von ca. 0.05 besitzen. Es erfolgt dann ein logarithmisches Wachstum bis zu einer OD 600 von 0,5-0,8. Bei Erreichen dieser finalen OD 600 werden die Zellen 81 mit einer 1 M Isopropyl-ß-D-thiogalactopyranosid (IPTG) - Lösung in einer Verdünnung von 1:1000 induziert. Nach Induktion wurde umgehend die Temperatur von 37 °C auf 20-15°C gesenkt und es erfolgte eine Expressionszeit von 4 h bis 24 h. Im Anschluss an die Expression wurden die Zellen in einen dementsprechenden Lysisbuffer (20mM Tris HCI pH 8.0, 150mM NaCl, 0,2% 10 mM NP-40 Imidazol) aufgenommen, resuspendiert und bei -20 °C bis -80°C gelagert. Durch gleichzeitige Zugabe eines Proteasehemmers (z. B. Complete EDTA free) wurde der proteolytische Abbau des rekombinanten Proteins durch E. coli-eigene Protease verhindert. Zur Überprüfung der Expression wurden ganze Zellen 81 vor und nach der Expression in SDS-Probenpuffer lysiert und das Lysat wurde in einem denaturierenden 15% SDS-Gel (SDS-PAGE) elektrophoretisch aufgetrennt.

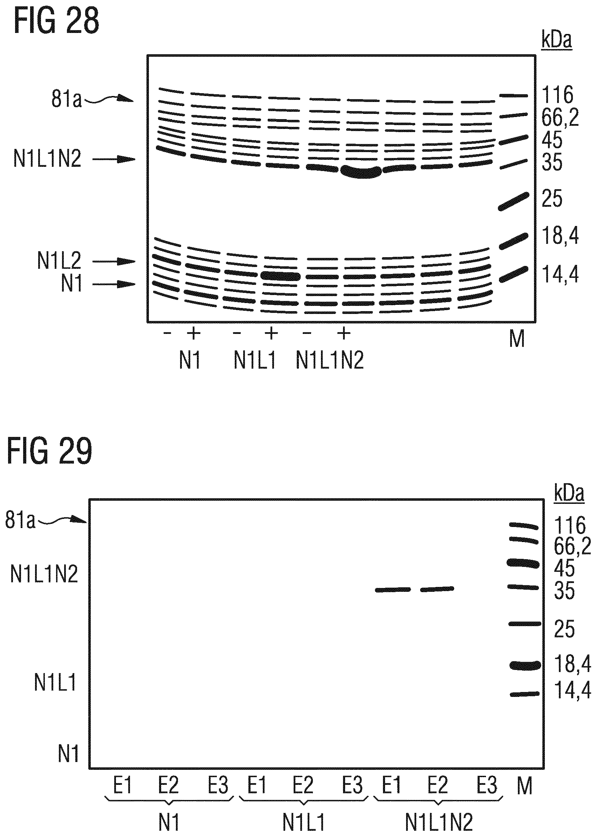

Fig. 28 zeigt die Überprüfung der Expression der G3P-Domänen (N1, N1L1 sowie N1 L2N2) in einem denaturierenden 15% SDS-Gel (SDS-PAGE), wobei - nicht induzierte Zellen,+ induzierte Zellen und M den Proteinmarker in kDa kennzeichnet. Die Pfeile kennzeichnen das rekombinante Protein der G3P-Domänen N1, N1L1 und N1 L2N2. Die zweite bis vierte Spalte von rechts beinhalten Negativkontrollen.

Nachfolgend werden Löslichkeitstest und Reinigung der G3P-Domänen N1, N1L1 und N1 L1 N2 erläutert.

Es kann vorkommen, dass nach der Expression die rekombinanten Proteine unlöslich in sogenannten Einschlusskörpern (inclusion bodies) vorliegen. Daher ist es bevorzugt, die exprimierten Proteine auf Ihre Löslichkeit zu testen. Aus praktischen Gründen wird dieser Löslichkeitstest gleichzeitig mit der Proteinreinigung verbunden, somit können mehrere Fragen in Bezug auf Reinheit, Proteinkonzentration und Degradation parallel abgeklärt werden. Für den Löslichkeitstest werden die Zellen 81 zunächst aufgetaut, dann erfolgt der Zellaufschluss, insbesondere über Ultraschall mit einer Sonotrode. Als erster Reinigungsschritt werden dann die aufgeschlossen Zellen 81 zentrifugiert (10.000 - 50.000 G). Sollte das exprimierte rekombinante Protein unlöslich als "Inclusion bodies" vorliegen, so wäre das Protein in dem Niederschlag zu finden. Befindet sich das Protein umgekehrt im Überstand, so liegt das Protein in löslicher Form vor, was in den meisten Fällen wünschenswert ist. Der nach der Zentrifugation entstandene Proteinrohextrakt 81 a wird dann, sollte es sich um ein His-Tag-Protein handeln, auf eine Nickel-Chelat-Säule (Nickel-NTA 31, Affinitätschromatographie) gegeben, wobei die rekombinanten G3P-Domänen über ihren His-Tag an dem Nickel-Chelat binden. Es erfolgen mehre Waschschritte mit unterschiedlichen Waschpuffern:
Waschschritt 1: 20 bis 50faches Bettvolumen, 20mM Tris, HCl pH 8.0, 150mM NaCl, 0,2% NP-40, 10mM Imidazol.
Waschschritt 2: 20 bis 100faches Bettvolumen, 20mM Tris, HCl pH 8.0, 150mM NaCl, 10mM Imidazol.
Waschschritt 3: 20 bis 100faches Bettvolumen, 20mM Tris, HCl pH 8.0, 1 M NaCl, 10mM Imidazol.
Waschschritt 4: 20 bis 100faches Bettvolumen, 20mM Tris, HCl pH 8.0, 150mM NaCl, 30mM Imidazol.
Waschschritt 5: 100 bis 50faches Bettvolumen, 20mM Tris, HCl pH 8.0, 150mM NaCl, 10mM Imidazol.

Zum Abschluss werden dann die so gereinigten rekombinanten G3P-Domänen mit einfachem oder ½-fachem Bettvolumen Elutionspuffer (20mM Tris, HCI pH 8.0, 150mM NaCl, 300mM Imidazol) von der Nickel-Chelat-Säule eluiert. Zur Überprüfung der Reinigung wir das eluierte rekombinante Protein, in diesem Fall die G3P-Domänen, auf einem SDS-Gel (SDS-PAGE) elektrophoretisch aufgetrennt. Das so entstandene rekombinante Protein kann nun durch Umsalzung oder Dialyse in einen Bindungspuffer überführt werden und steht für den Aufbau eines "Hybrid-Phago-Immuno-Assay" (HYPIA) oder eine spezifischen Anreicherung von E. coli zur Verfügung. Bei der Reinigung des rekombinaten His-Tag-Proteins entsteht ein Vorteil in Bezug auf die Assayentwicklung. Da das rekombinante Protein schon an den Beads (Ni-NTA 31, oder magnetischen Ni-Beads 26) über den His-Tag gebunden ist, können diese umgehend zur Anreicherung oder Assayentwicklung verwendet werden. Es ist also nicht mehr notwendig, das rekombinante Protein von den Beads zu eluieren.

Fig. 29 zeigt die Überprüfung der gereinigten rekombinanten G3P-Domänen auf ihre Löslichkeit und Reinheit. Die Spalten E1 - E3 zeigen die jeweilige Elution 1 bis 3 von dem Nickel-Chelat. N1 ist als kleinste G3P-Domäne unlöslich, es ist keine spezifischen Proteinbande erkennbar. N1L1als mittlere G3P-Domäne ist löslich, es ist deutlich eine Proteinbande bei ca. 14,4 kDa erkennbar. N1 L1 N2 als größte G3P-Domäne ist löslich, es ist deutlich eine Proteinbande bei ca. 35 kDa erkennbar.

Im Nachfolgenden wird eine Klonierungstrategie von Fusionsproteinen 50, bestehend aus g3p-Konstrukten und Reporterenzymen, in einen T7-basierenden Expressionsvektor erläutert.

Zur Herstellung eines Fusionsproteins 50 bestehend aus dem Bakteriophagenprotein G3P und einem Reporterenzym (GFP, EmGFP (SEQ ID NO 3, SEQ ID NO 4), Caspase-3 70 (SEQ ID NO 5, SEQ ID NO 6) oder Luziferase 71 (SEQ ID NO 7, SEQ ID NO 8)) ist es vorteilhaft, wenn das N-terminal liegende Protein kein Stopcodon besitzt, da die Proteinbiosynthese von N-terminal nach C-terminal verläuft und diese vorzugsweise nicht unterbrochen werden sollte. Desweiteren ist es bevorzugt, wenn das Reportergen (Gen des Reporterenzyms) mit anderen Restriktionenzymen kloniert wird als das G3P-Gen (Bakteriophagenprotein 12, Fängerprotein bzw. Captureprotein), da bei Verwendung gleicher Restriktionsenzyme ein serielles Klonieren erschwert ist. In dem hier vorgestellten Beispiel wurden die Reportegene EmGFP und Caspase-3 70 C-terminal hinter die g3p-Domänen N1L1 sowie N1 L1 N2 ligiert. Für die Ligation wurden folgende Restriktionsendonukleasen verwendet:
g3p-Domänen (Captureprotein):
   5'-Ende: BsmBI
   3'-Ende: Acc651
Reporterenzyme (EmGFP, Caspase-3 70 und Luziferase 71):
   5'-Ende: Sall
   3'-Ende: Notl

Wie zuvor bei den den g3p-Domänen wurden die Reportergene mit Hilfe der PCR-Technik von bestehenden Vektoren amplifiziert. Folgende Primer wurden für die Amplifikation verwendet:

| Reportergen | Richtung | Name | Sequenz |
|---|---|---|---|
| EmGFP | F | EGFP/Sall/fw | |
| EmGFP | R | EGFP/Notl/rv | |
| Caspase-3 | F | Casp3/Sall/fw | |
| Caspase-3 | R | Casp3/Notl/rv | |
| Luziferase | F | Luci/Sall/fw | |
| Luziferase | R | Luci/Notl/rv | |

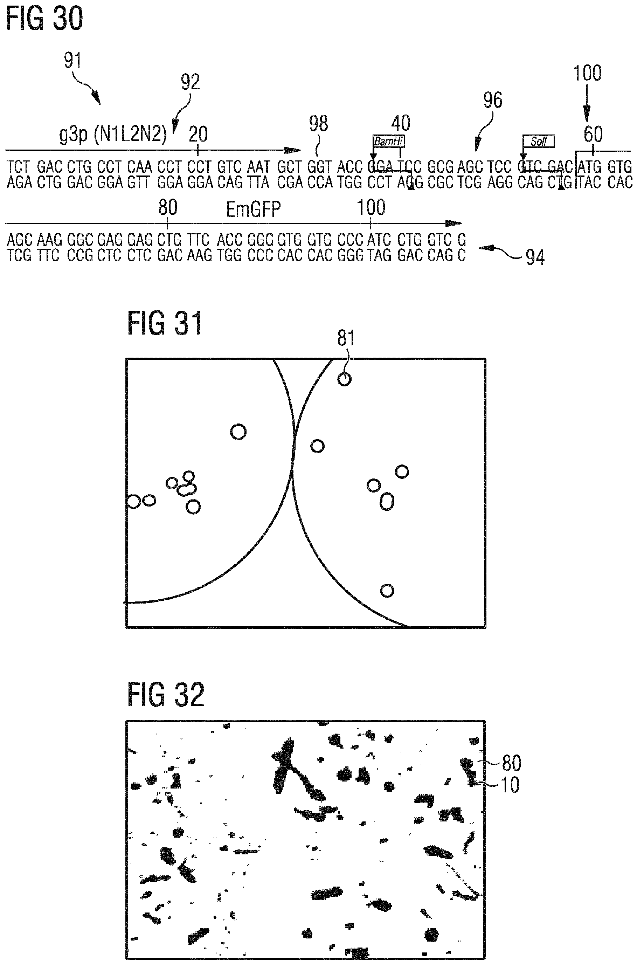

Die Klonierung der beiden Gene zu einem Fusionsprotein-Gen 91 erfolgte seriell, wobei die g3p-Domänen als Bakteriophagenprotein-Gen 92 als erstes ligiert wurden, gefolgt von den Reportergenen als Markerprotein-Gen 94. Nach der Ligation liegen das g3p-Gen N-terminal und das Reportergen C-terminal mit einem Linker 54 als Linkerprotein-Gen 96 von neun Codons 98 zwischen beiden Genen in einem T7-basierten Expressionsvektor vor, wie in Fig. 30 gezeigt, die eine Darstellung der Linkerregion zwischen der N-terminalen g3p-Domäne N1L1N2 und dem C-terminalen Reportergen EmGFP zeigt. Zwischen beiden Genen befindet sich ein Linker 54 mit neun Codons 98. Mit senkrechtem Pfeil dargestellt ist das Startcodon 100 (ATG) des Reportergens EmGFP.

Ein weiterer Aspekt bei der Generierung eines Fusionsproteins 50 ist die Tatsache, dass beide Gene vorzugsweise im gleichen Leserahmen liegen und dass sich bevorzugt kein Stopcodon im Linker 54 zwischen den beiden Genen befindet. Das gesamte Konstrukt wird dann nach einer Butanolfällung in E. coli NEB 5-alpha mittels Elektroporation transformiert und die Zellen 81 werden auf Nährböden mit 50 µg/ml Kanamycin selektiert. Die erhaltenen Transformanden werden nach einer DNA-Miniplasmidpräparation sowohl auf die Existenz der g3p-Domänen als auch auf die der Reportergene untersucht. Diese Analyse der Klone auf Ihre Richtigkeit erfolgt sowohl mit PCR als auch mit einem Restriktionsverdau.

Nachfolgend wird die Expression des Fusionsproteins 50 bestehend aus g3p-Domänen und Reporterenzymen (EmGFP und Caspase-3 70) erläutert.

Die Expression der Fusionsproteine 50 erfolgt unter den gleichen Bedingungen wie zuvor die reinen G3P-Domänen. Bei der Verwendung des EmGFP als Reporterprotein ist es bereits möglich, das Gelingen der Expression direkt zu beobachten. Auf Grund einer basalen Expression fluoreszieren die Zellen 81 bzw. Kolonien auf den Kulturplatten bei einer Exposition mit UV-Licht von 312 nm, wie in Fig. 31 gezeigt, die eine Überprüfung der Expression des G3P-N1L1N2 und EmGFP als Fusionsprotein 50 auf einem UV-Tisch bei einer Wellenlänge von 312 nm zeigt. Rechts sind Kolonien und eine Negativkontrolle einer reinen G3P-Domäne (N1L1N2) ohne EmGFP zu sehen, links Kolonien basaler Expression des Fusionsprotein N1 L1 N2-EmGFP; die Kolonien erscheinen hell.

Auch eine Analyse der fluoreszierenden E. coli BL21 (DE3) unter dem Fluoreszenzmikroskop bei einer Exzitation von 487 nm ist möglich. Hier erscheinen die Bakterien 10, wie in Fig. 32 zu sehen, hell bei einem dunklen Hintergrund. Fig. 32 zeigt demnach eine Analyse der fluoreszierenden E. coli BL21 (DE3) nach Expression des Fusionsproteins N1 L1 N2-EmGFP. Die Bakterien 10 erscheinen bei einer 400-fachen Vergrößerung hell bei einem dunklen Hintergrund.

Nachfolgend werden Löslichkeitstest und Reinigung des Fusionsproteins 50, bestehend aus g3p-Domänen und Reporterenzymen EmGFP, beschrieben.

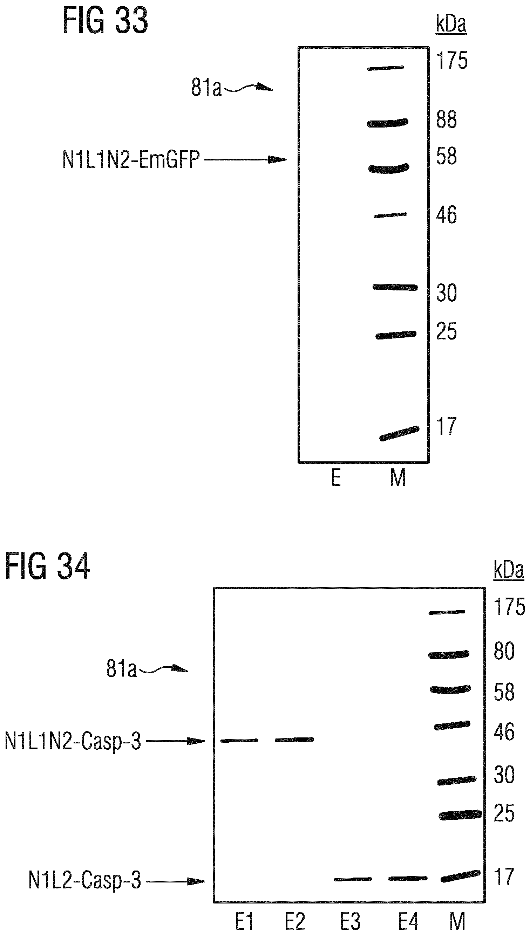

Der Löslichkeitstest sowie die Reinigung der Fusionsproteine 50 erfolgte nach dem gleichen Protokoll wie die reinen G3P-Domänen. Die Löslichkeit des Fusionsproteins 50 wurde in einem denaturierenden 15% SDS-Gel (SDS-PAGE) analysiert, wie in Fig. 33 und Fig. 34 gezeigt.

Fig. 33 zeigt die Überprüfung von Löslichkeitstest und Reinigung des Fusionsproteins N1L1N2-EmGFP im 15% SDS-Gel (SDS-PAGE), wobei E die Elution des Fusionsprotein N1L1N2-EmGFP von dem Nickel-Chelat und M den Proteinmarker in kDA 175 bis 17 kDa kennzeichnet. Der Pfeil markiert die Proteinbande des Fusionsproteins N1L1 N2-EmGFP mit ca. 60kDa.

Fig. 34 zeigt die Überprüfung von Löslichkeitstest und Reinigung der Fusionsproteine N1L1N2-Caspase-3 und N1L1-Caspase-3 im 15% SDS-Gel (SDS-PAGE). E1 kennzeichnet die erste Elution des Fusionsproteins N1L1N2- Caspase-3 von dem Nickel-Chelat, E2 kennzeichnet die zweite Elution des Fusionsproteins N1L1 N2-Caspase-3 von dem Nickel-Chelat, E3 kennzeichnet die erste Elution des Fusionsproteins N1 L1-Caspase-3 von dem Nickel-Chelat, E4 kennzeichnet die zweite Elution des Fusionsproteins N1 L1-Caspase-3 von dem Nickel-Chelat, M kennzeichnet den Proteinmarker in kDA 175 bis 17 kDa, die Pfeile markieren die Proteinbanden der Fusionsproteine N1L1N2-Caspase3 (ca. 42kDa) und N1L1-Caspase3 (17kDa).

Im Folgenden wird eine Bindungsstudie der G3P-Domäne N1L1N2L2-C an E. coli (NEB-alpha F'/q) in Form eines Pull-Down-Experiments erläutert.

In weiteren Experimenten konnte die längste G3P-Domäne N1L1N2L2-C erfolgreich in E. coli BL21 (DE3) löslich exprimiert werden. Ausgehend von dieser Tatsache wurde dieses rekombinierte Protein für Bindungsstudien in Form eines Pull-Down-Experiments bzw. Pull-Down-Assays verwendet. Das Pull-Down-Experiment ist ein Test, in dem eine Bindung zwischen zwei Proteinen (Protein-Protein Interaktion) nachgewiesen wird. In den vorliegenden Ergebnissen wird die Bindung bzw. die Protein-Protein-Interaktion zwischen dem G3P-Protein (Bakteriophagenprotein 12) und dem tolA-Protein 84 (E. coli) gezeigt. Das Prinzip des Pull-Down-Assays basiert auf der Affinität des bait-Moleküls (E. coli mit tolA-Protein 84) zu seinem prey-Molekül (G3P-Domäne N1L1N2L2-C und HisTag), das an einer Matrix (Ni-NTA 31) gebunden ist. Bei dem eigentlichen Test wird das bait-Molekül, in diesem Fall E. coli, mit tolA über die Matrix (Ni-NTA 31 und N1 L1 N2L2-C mit His-Tag) gebunden. Nach der Bindung der beiden Interaktionspartner (G3P - tolA) werden dann alle nicht gebundenen Proteine durch intensives Waschen von der Matrix entfernt. Abschließend wird dann der Protein-Protein-Komplex von der Matrix eluiert und in einem SDS- Gel analysiert. In Bezug auf die finale Analyse der G3P-tolA-Interaktion wurde der hier vorgestellte klassische Pull-Down-Assay jedoch etwas modifiziert. In den vorliegenden Ergebnissen wurde nicht der eluierte Proteinkomplex im SDS-Gel analysiert, sondern der Durchlauf der auf die Matrix gegebenen Bakterienkonzentration. Experimentell wurde auf der mit G3P-Protein (N1 L1 N2L2-C und His-Tag) beschichteten Matrix (Ni-NTA 31) eine definierte Bakterienkonzentration (E. coli, Stamm NEB 5-alpha F'/q) von 2,4E+06 Zellen 81 gegeben. Nach Durchlaufen der mit G3P-beschichteten Ni-Matrix reduzierte sich die ursprüngliche Bakterienkonzentration auf 1,1E+06 Zellen 81, wobei 1,3E+06 Zellen 81 an der Matrix über das G3P-Protein gebunden waren. Rechnerisch wurden also 54,2 % von der ursprünglich eingesetzten Bakterienkonzentration (2,4E+06 E. coli) mit Hilfe der G3P- Domäne (N1 L1 N2L2-C) gebunden. Als Negativkontrolle diente Ni-NTA 31, das nicht mit der G3P- Domäne (N1 L1 N2L2-C) beschichtet wurde.

Es zeigte sich, dass im Falle der Negativkontrolle keine Bindung beobachtet werden konnte und im Durchlauf fast die ursprüngliche Bakterienkonzentration nachgewiesen wurde (2,8E+06 Zellen 81). Auf Grund der hier vorgestellten Ergebnisse konnte die Protein-Protein-Interaktion zwischen dem G3P-Bakteriophagenprotein 12 und dem bakteriellen tolA-Protein 84 (E coli) eindeutig nachgewiesen werden.

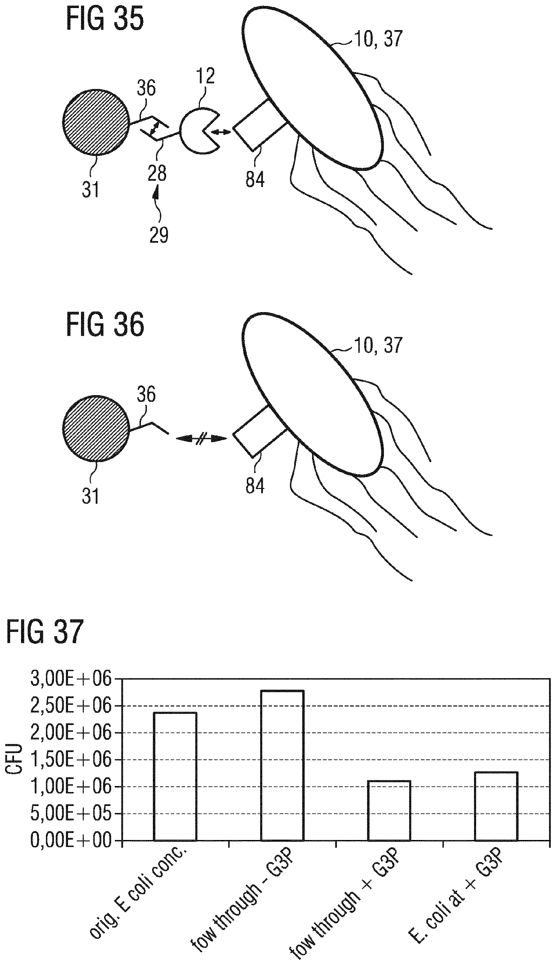

Figs. 35 und 36 zeigen jeweils eine schematische Darstellung eines Pull-Down-Experiments zum Nachweis der Protein-Protein-Interaktion zwischen dem Bakteriophagenprotein 12 G3P und dem bakteriellen Oberflächenprotein 32 tolA.

In Fig. 35 ist die Bindung zwischen G3P und tolA gezeigt, wobei Ni-NTA 31 als Matrix verwendet wird, an die Ni-Chelat und Ni-lonen das His-Tag des G3P, das Bakteriophagenprotein 12 G3P bzw. eine G3P-Domäne, über eine Protein-Protein-Interaktion zwischen G3P und tolA ein E. coli Oberflächenprotein tolA und damit ein E. coli mit peritricher Begeißelung gebunden ist.

Fig. 36 zeigt eine Negativkontrolle ohne das Bakteriophagenprotein 12 G3P, wobei keine Bindung an die Ni-NTA-Matrix zu beobachten ist.

Fig. 37 zeigt eine graphische Darstellung der Ergebnisse des Pull-Down-Experiments zum Nachweis der Protein-Protein-Interaktion zwischen dem Bakteriophagenprotein 12 G3P und dem bakteriellen Protein tolA. Analysiert wurde jeweils der Durchlauf einer definierten E. coli-Konzentration von 2,4E+06 (erster Balken von links). Der zweite Balken von links zeigt eine Negativkontrolle, d.h. den Durchlauf der Bakterienkonzentration an der Matrix ohne G3P (2,8E+06). Der dritte Balken von links zeigt die Bakterienkonzentration nach dem Durchlaufen über der Matrix mit G3P (1,1E+06). Der vierte Balken von links zeigt die Bakterienkonzentration, die an der Matrix haften geblieben ist (1,3E+06).

### Bezugszeichenliste:

- 10: Bakterium
- 12: Bakteriophagenprotein
- 14: Antikörper
- 16: Bakteriophagen
- 18: Bakterienoberfläche
- 19: Anreicherungsstruktur
- 19a: Oberflächenstruktur
- 20: magnetischer Bead
- 22: Biotin
- 24: Streptavidin
- 25: Biotin-Streptavidin-Linker
- 26: Nickel-Bead
- 28: Polyhistidin-Tag
- 29: Metallchelat-Polyhistidin-Tag-Linker
- 30: nichtmagnetischer Bead
- 31: Ni-NTA
- 31a: Chromatographiesäule
- 31b: Sepharose
- 31c: Mikrotiterplatte
- 31d: Kunststoff
- 31e: Filtermaterial
- 31f: Glas
- 31g: Metall
- 31h: Keramik
- 31i: Holz
- 31j: Gestein
- 31k: Gewebe
- 31l: Nanopartikel
- 31m: Halbleitermaterial
- 31n: Linkerstruktur
- 32: bakterielles Oberflächenprotein
- 34: Metallchelat-Bead
- 36: Metallchelat
- 37: Bakterienfamilie
- 38: Sepharosekügelchen
- 40: Materialoberfläche
- 42: Detektionsprotein
- 43: Detektionsproteinoberfläche
- 44: Markierung
- 45: Markermolekül
- 46: Protein A
- 48: Protein G
- 49: Fluoreszenzmolekül
- 50: Fusionsprotein
- 52: Enzym
- 54: Markerprotein
- 56: Linkerprotein
- 58: Reinigungs-Tag
- 60: Proteaseschnittstelle
- 62: N-Terminus
- 64: C-Terminus
- 66: Proteaseerkennungssequenz
- 67: Protein
- 68: humane Caspase
- 70: Caspase-3
- 71: Luziferase
- 72: fluoreszierendes Protein
- 74: Polymerase
- 75: Tag-Polymerase
- 76: lytisches Protein
- 78: lytisches Bakteriophagenprotein
- 80: Organismus
- 81: Zelle
- 81a: Proteinextrakt
- 82: F-Pilus
- 83: äußere Membran
- 84: tolA-Protein
- 86: periplasmatischer Raum
- 88: Cytoplasmamembran
- 90: Restriktionsenzym
- 91: Fusionsprotein-Gen
- 92: Bakteriophagenprotein-Gen
- 94: Markerprotein-Gen
- 96: Linkerprotein-Gen
- 98: Codon
- 100: Startcodon
- N1: Proteindomäne
- N2: Proteindomäne
- L1: Proteindomäne
- L2: Proteindomäne
- TD: Transmembrandomäne
- M13: Bakteriophagen
- G3P: Protein
- G3p: Gen
- M: DNA-Marker
- E: Elution
- EmGFP: Protein

<110> EADS Deutschland GmbH; Astrium GmbH
<120> Detektionsverfahren zur Detektion von Bakterien, verfahren zur Herstellung von Fusionsproteinen und Fusionsprotein
<160> 8
<210> SEQ ID NO 1
   <211> 1275
   <212> DNA
   <213> Bakteriophage M13
<400>
<110> EADS Deutschland GmbH; Astrium GmbH
<120> Detektionsverfahren zur Detektion von Bakterien, verfahren zur Herstellung von Fusionsproteinen und Fusionsprotein
<160> 8
<210> SEQ ID NO 2
   <211> 424
   <212> DNA
   <213> Bakteriophage M13
<400>
<110> EADS Deutschland GmbH; Astrium GmbH
<120> Detektionsverfahren zur Detektion von Bakterien, verfahren zur Herstellung von Fusionsproteinen und Fusionsprotein
<160> 8
<210> SEQ ID NO 3
   <211> 720
   <212> DNA
   <213> unbekannt
<400>
   ATGGTGAGCAAGGGCGAGGAGCTGTTCACCGGGGTGGTGCCCATCCTGGTCGAGCTGGAC
   GGCGACGTAAACGGCCACAAGTTCAGCGTGTCCGGCGAGGGCGAGGGCGATGCCACCTAC
   GGCAAGCTGACCCTGAAGTTCATCTGCACCACCGGCAAGCTGCCCGTGCCCTGGCCCACC
   CTCGTGACCACCTTGACCTACGGCGTGCAGTGCTTCGCCCGCTACCCCGACCACATGAAG
   CAGCACGACTTCTTCAAGTCCGCCATGCCCGAAGGCTACGTCCAGGAGCGCACCATCTTC
   TTCAAGGACGACGGCAACTACAAGACCCGCGCCGAGGTGAAGTTCGAGGGCGACACCCTG
   GTGAACCGCATCGAGCTGAAGGGCATCGACTTCAAGGAGGACGGCAACATCCTGGGGCAC
   AAGCTGGAGTACAACTACAACAGCCACAAGGTCTATATCACCGCCGACAAGCAGAAGAAC
   GGCATCAAGGTGAACTTCAAGACCCGCCACAACATCGAGGACGGCAGCGTGCAGCTCGCC
   GACCACTACCAGCAGAACACCCCCATCGGCGACGGCCCCGTGCTGCTGCCCGACAACCAC
   TACCTGAGCACCCAGTCCGCCCTGAGCAAAGACCCCAACGAGAAGCGCGATCACATGGTC
   CTGCTGGAGTTCGTGACCGCCGCCGGGATCACTCTCGGCATGGACGAGCTGTACAAGTAA
<110> EADS Deutschland GmbH; Astrium GmbH
<120> Detektionsverfahren zur Detektion von Bakterien, verfahren zur Herstellung von Fusionsproteinen und Fusionsprotein
<160> 8
<210> SEQ ID NO 4
   <211> 240
   <212> DNA
   <213> unbekannt
<400>
<110> EADS Deutschland GmbH; Astrium GmbH
<120> Detektionsverfahren zur Detektion von Bakterien, verfahren zur Herstellung von Fusionsproteinen und Fusionsprotein
<160> 8
<210> SEQ ID NO 5
   <211> 834
   <212> DNA
   <213> unbekannt
<400>
<110> EADS Deutschland GmbH; Astrium GmbH
<120> Detektionsverfahren zur Detektion von Bakterien, verfahren zur Herstellung von Fusionsproteinen und Fusionsprotein
<160> 8
<210> SEQ ID NO 6
   <211> 277
   <212> DNA
   <213> unbekannt
<400>
<110> EADS Deutschland GmbH; Astrium GmbH
<120> Detektionsverfahren zur Detektion von Bakterien, verfahren zur Herstellung von Fusionsproteinen und Fusionsprotein
<160> 8
<210> SEQ ID NO 7
   <211> 1653
   <212> DNA
   <213> unbekannt
<400>
   atggaagacgccaaaaacataaagaaaggcccggcgccattctatcctctagaggatgga
   accgctggagagcaactgcataaggctatgaagagatacgccctggttcctggaacaatt
   gcttttacagatgcacatatcgaggtgaacatcacgtacgcggaatacttcgaaatgtcc
   gttcggttggcagaagctatgaaacgatatgggctgaatacaaatcacagaatcgtcgta
   tgcagtgaaaactctcttcaattctttatgccggtgttgggcgcgttatttatcggagtt
   gcagttgcgcccgcgaacgacatttataatgaacgtgaattgctcaacagtatgaacatt
   tcgcagcctaccgtagtgtttgtttccaaaaaggggttgcaaaaaattttgaacgtgcaa
   aaaaaattaccaataatccagaaaattattatcatggattctaaaacggattaccaggga
   tttcagtcgatgtacacgttcgtcacatctcatctacctcccggttttaatgaatacgat
   tttgtaccagagtcctttgatcgtgacaaaacaattgcactgataatgaattcctctgga
   tctactgggttacctaagggtgtggcccttccgcatagaactgcctgcgtcagattctcg
   catgccagagatcctatttttggcaatcaaatcattccggatactgcgattttaagtgtt
   gttccattccatcacggttttggaatgtttactacactcggatatttgatatgtggattt
   cgagtcgtcttaatgtatagatttgaagaagagctgtttttacgatcccttcaggattac
   aaaattcaaagtgcgttgctagtaccaaccctattttcattcttcgccaaaagcactctg
   attgacaaatacgatttatctaatttacacgaaattgcttctgggggcgcacctctttcg
   aaagaagtcggggaagcggttgcaaaacgcttccatcttccagggatacgacaaggatat
   gggctcactgagactacatcagctattctgattacacccgagggggatgataaaccgggc
   gcggtcggtaaagttgttccattttttgaagcgaaggttgtggatctggataccgggaaa
   acgctgggcgttaatcagagaggcgaattatgtgtcagaggacctatgattatgtccggt
   tatgtaaacaatccggaagcgaccaacgccttgattgacaaggatggatggctacattct
   ggagacatagcttactgggacgaagacgaacacttcttcatagttgaccgcttgaagtct
   ttaattaaatacaaaggatatcaggtggcccccgctgaattggaatcgatattgttacaa
   caccccaacatcttcgacgcgggcgtggcaggtcttcccgacgatgacgccggtgaactt
   cccgccgccgttgttgttttggagcacggaaagacgatgacggaaaaagagatcgtggat
   tacgtcgccagtcaagtaacaaccgcgaaaaagttgcgcggaggagttgtgtttgtggac
   gaagtaccgaaaggtcttaccggaaaactcgacgcaagaaaaatcagagagatcctcata
   aaggccaagaagggcggaaagtccaaattgtaa
<110> EADS Deutschland GmbH; Astrium GmbH
<120> Detektionsverfahren zur Detektion von Bakterien, verfahren zur Herstellung von Fusionsproteinen und Fusionsprotein
<160> 8
<210> SEQ ID NO 8
   <211> 550
   <212> DNA
   <213> unbekannt
<400>

## Patentansprüche

1. Detektionsverfahren zur Detektion von Bakterien (10), aufweisend die Schritte
a) spezifisches Anreichern eines bakteriellen Oberflächenproteins (32) an einer Anreicherungsstruktur (19);
b) spezifische Detektionsreaktion eines Detektionsproteins (42) mit dem bakteriellen Oberflächenprotein (32);
c) Erfassen des Detektionsproteins (42),
wobei als Detektionsprotein (42) ein Fusionsprotein (50) verwendet wird, das durch Verbinden eines Bakteriophagenproteins (12) mit einem Markerprotein (54) über ein Linkerprotein (56) hergestellt wird, wobei das Markerprotein (54) aus der Gruppe der humanen Caspasen (68) ausgewählt wird,
wobei die spezifische Detektionsreaktion durch spezifische Protein-Protein-Interaktion des bakteriellen Oberflächenproteins (32) mit dem Bakteriophagenprotein (12) erfolgt.

2. Verfahren nach Anspruch 1,
**dadurch gekennzeichnet, dass** ein freies bakterielles Oberflächenprotein (32) oder ein gebundenes, insbesondere ein an ein Bakterium (10) gebundenes, bakterielles Oberflächenprotein (32) angereichert wird, wobei die Anreicherungsstruktur (19) insbesondere an einer immobilen oder einer mobilen Oberflächenstruktur (19a) bereitgestellt wird.

3. Verfahren nach Anspruch 2,
**dadurch gekennzeichnet, dass** die Anreicherungsstruktur (19) über eine Linker-Struktur (31 n), insbesondere über einen Biotin-Streptavidin-Linker (25) oder einen Metallchelat-Polyhistidin-Tag-Linker (29), an der Oberflächenstruktur (19a) immobilisiert wird, wobei als Oberflächenstruktur (19a) insbesondere magnetische Beads (20), mehr insbesondere Ni-Beads (26), nichtmagnetische Beads (30), insbesondere Ni-NTA (31), Chromatographiesäulenmaterialien (31 a), Sepharosen (31 b), Mikrotiterplatten (31 c), Kunststoffe (31 d), Filteroberflächen (31 e), Glas (31 f), Metall (31 g), Keramik (31 h), Holz (31i), Gestein (31j), Gewebe (31 k), Nanopartikel (31l) und/oder Halbleitermaterialien (31 m) verwendet werden.

4. Verfahren nach einem der Ansprüche 1 bis 3,
**dadurch gekennzeichnet, dass** sowohl das Anreichern als auch die Detektionsreaktion durch spezifische Protein-Protein-Interaktion mit einem bakteriellen Oberflächenprotein (32) erfolgt, wobei als Detektionsprotein (42) das Fusionsprotein (50) und als Anreicherungsstruktur (19) ein Antikörper (14) verwendet wird.

5. Verfahren nach einem der Ansprüche 1 bis 4,
**dadurch gekennzeichnet, dass** das Fusionsprotein (50) insbesondere über eine Proteaseschnittstelle (60) mit einem Reinigungs-Tag (58) gekoppelt wird.

6. Verfahren nach einem der Ansprüche 1 bis 5,
**dadurch gekennzeichnet, dass** das Fusionsprotein (50) durch rekombinante Expression hergestellt wird.

7. Verfahren nach einem der Ansprüche 1 bis 6,
**dadurch gekennzeichnet, dass** vor Schritt a) ein Proteinherstellungsschritt mit den folgenden Schritten durchgeführt wird:
i) rekombinantes Exprimieren eines Fusionsproteins (50) oder eines Bakteriophagenproteins (12) in einem niederen prokaryotischen oder eukaryotischen Organismus (80);
ii) Aufschließen der Zellen (81) des niederen prokaryotischen oder eukaryotischen Organismus (80);
iii) Bilden eines Proteinextrakts (81a);
iv) Reinigen des Proteinextrakts (81a),
wobei insbesondere die durch die Expression gebildeten Fusionsproteine (50) stabilisiert werden, insbesondere durch Vakuumtrocknen, Gefriertrocknen, Lyophilisieren, Sublimationstrocknen, PEGyliseren, Protein-Engineering und/oder durch Zugeben von Stabilisatoren.

8. Verfahren zur Herstellung von Fusionsproteinen (50) mit den Schritten
A) Klonieren eines Fusionsproteingens (91);
B) Transformieren des Fusionsproteingens (91) in einen niederen prokaryotischen oder eukaryotischen Organismus (80);
C) Exprimieren von Fusionsproteinen (50) in dem Organismus (80);
D) Reinigen der Fusionsproteine (50),
wobei in Schritt A) ein Bakteriophagenprotein-Gen (92), ein Linkerprotein-Gen (96) und ein Markerprotein-Gen (94) ligiert werden, wobei als Markerprotein-Gene (94) Gene zur Bildung von humanen Caspasen (68) verwendet werden.

9. Verfahren nach Anspruch 8,
**dadurch gekennzeichnet, dass** in Schritt A) die Ligation seriell durchgeführt wird, wobei insbesondere zuerst das Bakteriophagenprotein-Gen (92), insbesondere N-terminal, und dann das Markerprotein-Gen (94), insbesondere C-terminal, mit dem Linkerprotein-Gen (96) ligiert wird.

10. Verfahren nach einem der Ansprüche 8 oder 9,
**dadurch gekennzeichnet, dass** für die Ligation unterschiedliche Restriktionsenzyme (90) für das Bakteriophagenprotein-Gen (92) und das Markerprotein-Gen (94) verwendet werden, insbesondere BsmBI als 5'-Ende und Acc65I als 3'-Ende für das Bakteriophagenprotein-Gen (92) und Sall als 5'-Ende und Notl als 3'-Ende für das Markerprotein-Gen (94).

11. Verfahren nach einem der Ansprüche 8 bis 10,
**dadurch gekennzeichnet, dass** ein Linkerprotein (56) erzeugt wird, das eine freie dreidimensionale Bewegung des Bakteriophagenproteins (12) und des Markerproteins (54) zueinander ermöglicht, wobei das Linkerprotein (56) insbesondere eine Länge von neun Codons (98) aufweist und mehr insbesondere frei von Stopcodons ist.

12. Fusionsprotein (50), erhältlich mit einem Verfahren nach einem der Ansprüche 8 bis 11.

## Claims

1. A detection method for detecting bacteria (10), comprising the steps of
a) specifically enriching a bacterial surface protein (32) on an enrichment structure (19);
b) specific detection reaction of a detection protein (42) with the bacterial surface protein (32);
c) detecting the detection protein (42),
wherein a fusion protein (50), which is produced by bonding a bacteriophage protein (12) to a marker protein (54) via a linker protein (56), is used as the detection protein (42), the marker protein (54) being selected from the group of human caspases (68),
wherein the specific detection reaction takes place by a specific protein-protein interaction of the bacterial surface protein (32) with the bacteriophage protein (12).

2. The method according to claim 1,
**characterized in that** a free bacterial surface protein (32) or a bound bacterial surface protein (32), in particular one bound to a bacterium (10), is enriched, wherein the enrichment structure (19) is provided in particular on an immobile or a mobile surface structure (19a).

3. The method according to claim 2,
**characterized in that** the enrichment structure (19) is immobilized on the surface structure (19a) via a linker structure (31 n), in particular via a biotin-streptavidin linker (25) or a metal-chelate polyhistidine tag linker (29), wherein in particular magnetic beads (20) more particularly Ni beads (26), non-magnetic beads (30), in particular Ni-NTA (31), chromatography column materials (31 a), Sepharoses (31 b), microtiter plates (31 c), plastics (31 d), filter surfaces (31 e), glass (31 f), metal (31 g), ceramics (31 h), wood (31 i), rock (31j), fabric (31k), nanoparticles (31l) and/or semiconductor materials (31 m) are used as the surface structure (19a).

4. The method according to any one of the claims 1 to 3,
**characterized in that** both the enrichment and the detection reaction take place by specific protein-protein interaction with a bacterial surface protein (32), wherein the fusion protein (50) is used as the detection protein (42) and an antibody (14) as the enrichment structure (19).

5. The method according to any one of the claims 1 to 4,
**characterized in that** the fusion protein (50) is coupled to a purification tag (58) particularly via a protease interface (60).

6. The method according to any one of the claims 1 to 5,
**characterized in that** the fusion protein (50) is produced by recombinant expression.

7. The method according to any one of the claims 1 to 6,
**characterized in that**, prior to step a), a protein production step with the following steps is carried out:
i) recombinant expression of a fusion protein (50) or a bacteriophage protein (12) in a lower prokaryotic or eukaryotic organism (80);
ii) disruption of the cells (81) of the lower prokaryotic or eukaryotic organism (80);
iii) forming a protein extract (81 a);
iv) purifying the protein extract (81 a);
wherein, in particular, the fusion proteins (50) formed by the expression are stabilized, in particular by vacuum drying, freeze drying, lyophilization, sublimation drying, PEGylation, protein engineering and/or addition of stabilizers.

8. A method for producing fusion proteins (50), comprising the steps of
A) cloning a fusion protein gene (91);
B) transforming a fusion protein gene (91) into a lower prokaryotic or eukaryotic organism (80);
C) expressing fusion proteins (50) in the organism (80);
D) purifying the fusion proteins (50),
wherein a bacteriophage protein gene (92), a linker protein gene (96) and a marker protein gene (94) are ligated in step A), with genes for forming human caspases (68) being used as the marker protein gene (94).

9. The method according to claim 8,
**characterized in that** in step A), the ligation is carried out in a serial manner, wherein, in particular, the bacteriophage protein gene (92) is ligated first, in particular N-terminally, and then, in particular C-terminally, the marker protein gene (94) with the linker protein gene (96).

10. The method according to any one of the claims 8 or 9,
**characterized in that** for ligation, different restriction enzymes (90) are used for the bacteriophage protein gene (92) and the marker protein gene (94), in particular BsmBl as a 5' end and Acc65l as a 3' end for the bacteriophage protein gene (92) and Sall as a 5' end and Notl as a 3' end for the marker protein gene (94).

11. The method according to any one of the claims 8 to 10,
**characterized in that** a linker protein (56) is produced that enables a free three-dimensional movement of the bacteriophage protein (12) and the marker protein (54) relative to each other, wherein the linker protein (56), in particular, has a length of nine codons (98) and, more particularly, is free from stop codons.

12. A fusion protein (50) obtainable with a method according to any one of the claims 8 to 11.

## Revendications

1. Procédé de détection de bactéries (10) comprenant des étapes consistant à :
a) effectuer un enrichissement spécifique d'une protéine de surface bactérienne (32) sur une structure d'enrichissement (19),
b) effectuer une réaction de détection spécifique d'une protéine de détection (42) avec la protéine de surface bactérienne (32),
c) détecter la protéine de détection (42),
procédé selon lequel en tant que protéine de détection (42) on utilise une protéine de fusion (50) obtenue par jonction d'une protéine bactériophage (12) et d'une protéine de marquage (54) par l'intermédiaire d'une protéine de jonction (56), la protéine de marquage (54) étant choisie dans le groupe des caspases humaines (68), et
la réaction de détection spécifique est effectuée par interaction protéine-protéine spécifique de la protéine de surface bactérienne (32) avec la protéine bactériophage (12).

2. Procédé conforme à la revendication 1,
**caractérisé en ce que**
l'on enrichit une protéine de surface bactérienne libre (32) ou une protéine de surface bactérienne (32) liée, en particulier liée à une bactérie (10), la structure d'enrichissement (19) étant en particulier préparée sur une structure de surface immobile ou mobile (19a).

3. Procédé conforme à la revendication 2,
**caractérisé en ce que**
la structure d'enrichissement (19) est immobilisée sur la structure de surface (19a) par l'intermédiaire d'une structure de jonction (31 n), en particulier un lieur biotine-streptavidine (25) ou un lieur étiqueté chélate métallique-polyhistidine-marque (29), la structure de surface (19a) étant en particulier des perles magnétiques (20), plus particulièrement des perles de Ni (26), des perles non magnétiques (30), en particulier Ni-NTA (31), des matériaux de colonnes de chromatographie (31a), des sé-pharoses (31b), des microplaques (31c), des matériaux synthétiques (31d), des surfaces filtrantes (31e), du verre (31f), du métal (31g), de la céramique (31h), du bois (31i), des roches (31j), des tissus (31k), des nanoparticules (311) et/ou des matériaux semi-conducteurs (31m).

4. Procédé conforme à l'une des revendications 1 à 3,
**caractérisé en ce que**
l'enrichissement et la réaction de détection sont effectué(s) par interaction protéine-protéine spécifique avec une protéine de surface bactérienne (32), la protéine de fusion (50) étant utilisée en tant que protéine de détection (42), et un anticorps (14) étant utilisé en tant que structure d'enrichissement (19).

5. Procédé conforme à l'une des revendications 1 à 4,
**caractérisé en ce que**
la protéine de fusion (50) est couplée à une étiquette de purification (58), en particulier par l'intermédiaire d'une interface de protéase (60).

6. Procédé conforme à l'une des revendications 1 à 5,
**caractérisé en ce que**
la protéine de fusion (50) est obtenue par expression recombinante.

7. Procédé conforme à l'une des revendications 1 à 6,
**caractérisé en ce qu'**
avant l'étape a), on met en oeuvre une étape d'obtention de protéine comprenant les étapes suivantes consistant à :
i) effectuer une expression recombinante d'une protéine de fusion (50) ou d'une protéine bactériophage (12) dans un organisme procaryote ou eurocaryote inférieur (80),
ii) ouvrir les cellules (81) de l'organisme procaryote ou eurocaryote inférieur (80),
iii) former un extrait de protéine (81a),
iv) purifier l'extrait de protéine (81a),
la protéine de fusion (50) formée par l'expression étant en particulier stabilisée, en particulier par séchage sous vide, séchage par réfrigération, lyophilisation, séchage par sublimation, PEGylation, traitement de protéine et/ou addition d'agents de stabilisation.

8. Procédé d'obtention d'une protéine de fusion (50) comprenant des étapes consistant à :
A) cloner un gène de protéine de fusion (91),
B) transformer le gène de protéine de fusion (91) en un organisme procaryote ou eurocaryote inférieur (80),
C) exprimer des protéines de fusion (50) dans l'organisme (80),
D) purifier les protéines de fusion (50),
dans l'étape A), un gène de protéine bactériophage (92), un gène de protéine de jonction (96) et un gène de protéine de marquage (94) étant ligaturés, et en tant que gène de protéine de marquage (94) des gènes permettant de former des caspases humaines (68) étant utilisés.

9. Procédé conforme à la revendication 8,
**caractérisé en ce que**
dans l'étape A), la ligature est effectuée en série et en particulier le gène de protéine bactériophage (92), en particulier son extrémité N terminale est tout d'abord ligaturé avec le gène de protéine de jontion (96) et ensuite le gène de protéine de marquage (94), en particulier son extrémité C-terminale est ligaturé avec le gène de protéine de jonction (96).

10. Procédé conforme à l'une des revendications 8 ou 9,
**caractérisé en ce que**
pour effectuer la ligature, on utilise des enzymes de restriction (90) différentes pour le gène de protéine bactériophage (92) et le gène de protéine de marquage (94), en particulier BsmBl en tant qu'extrémité 5' et Acc65l en tant qu'extrémité 3' pour le gène de protéine bactériophage (92) et Sall en tant qu'extrémité 5' et Notl en tant qu'extrémité 3' pour le gène de protéine de marquage (94).

11. Procédé conforme à l'une des revendications 8 à 10,
**caractérisé en ce que**
l'on obtient une protéine de jonction (56) permettant un déplacement tridimensionnel libre de la protéine bactériophage (12) et de la protéine de marquage (54) l'une par rapport à l'autre, la protéine de jonction (56) ayant en particulier une longueur de neuf codons (98) et de façon plus préférentielle étant exempte de codons stop.

12. Protéine de fusion (50) susceptible d'être obtenue par la mise en oeuvre du procédé conforme à l'une des revendications 8 à 11.
